# EUROPÄISCHE PATENTSCHRIFT

(11) **EP 1 520 013 B1**
(45) Veröffentlichungstag und Bekanntmachung des Hinweises auf die Patenterteilung: **01.09.2010**
(21) Anmeldenummer: 03763598.4
(22) Anmeldetag: 08.07.2003
(51) Int. Cl.: C12N 9/04, C12N 15/53, C12N 15/63

(54) **NUKLEOTIDSEQUENZEN CODIEREND FÜR DEREGULIERTE PHOSPHOGLYCERAT-DEHYDROGENASEN CORYNEFORMER BAKTERIEN SOWIE VERFAHREN ZUR HERSTELLUNG VON L-SERIN**
NUCLEOTIDE SEQUENCES THAT ENCODE DEREGULATED PHOSPHOGLYCERATE DEHYDROGENASES OF CORYNEFORM BACTERIA AND METHOD FOR PRODUCING L-SERINE
SEQUENCES NUCLEOTIDIQUES CODANT POUR DES PHOSPHOGLYCERATE-DESHYDROGENASES DEREGULEES DE BACTERIES CORYNEFORMES ET PROCEDE DE PRODUCTION DE L-SERINE

(30) Priorität: 10.07.2002 DE 10231297
(43) Veröffentlichungstag der Anmeldung: 06.04.2005
(73) Patentinhaber: Forschungszentrum Jülich GmbH, 52425 Jülich (DE); AMINO GmbH, D-38373 Frellstedt (DE)
(72) Erfinder: EGGELING, Lothar, 52428 Jülich (DE); PETERS-WENDISCH, Petra, 52428 Jülich (DE); NETZER, Roman, 66346 Püttlingen (DE); SAHM, Hermann, 52428 Jülich (DE); FAURIE, Robert, 38154 Königslutter (DE); KLASSEN, Birgit, 38108 Braunschweig (DE)
(86) Internationale Anmeldenummer: PCT/DE2003/002290
(87) Internationale Veröffentlichungsnummer: WO 2004/007705

(56) Entgegenhaltungen:
- EP-A- 0 943 687
- US-A- 5 624 828
- US-B1- 6 180 373
- ARCHER J A C ET AL: "A C-TERMINAL DELETION IN CORYNEBACTERIUM-GLUTAMICUM HOMOSERINE DEHYDROGENASE ABOLISHES ALLOSTERIC INHIBITION BY L THREONINE" GENE (AMSTERDAM), Bd. 107, Nr. 1, 1991, Seiten 53-60, XP001155222 ISSN: 0378-1119
- PETERS-WENDISCH P ET AL: "3-Phosphoglycerate dehydrogenase from Corynebacterium glutamicum: The C-terminal domain is not essential for activity but is required for inhibition by L-serine." APPLIED MICROBIOLOGY AND BIOTECHNOLOGY, Bd. 60, Nr. 4, 20. Dezember 2002 (2002-12-20), Seiten 437-441, XP002255644 ISSN: 0175-7598
- BELL JESSICA K ET AL: "De-regulation of D-3-phosphoglycerate dehydrogenase by domain removal." EUROPEAN JOURNAL OF BIOCHEMISTRY, Bd. 269, Nr. 17, September 2002 (2002-09), Seiten 4176-4184, XP002255645 =ejb&page=aims September, 2002 ISSN: 0014-2956

## Beschreibung

Die Erfindung betrifft Nukleotidsequenzen coryneformer Bakterien codierend für an der Biosynthese von L-Serin beteiligte Proteine sowie Verfahren zur Herstellung von L-Serin.

Die Aminosäure L-Serin findet in der Nahrungsmittel-, Futtermittel- und Pharmaindustrie, sowie in der Humanmedizin Anwendung. Darüber hinaus dient sie als Baustein für die Synthese weiterer industriell verwertbarer Produkte, wie z. B. L-Tryptophan aus Indol und L-Serin.

Es ist bekannt, dass L-Serin durch Fermentation von Stämmen coryneformer Bakterien hergestellt werden kann. So ist z. B. ein Stamm von *Corynebacterium glycinophilum* in der Lage, L-Serin aus Glycin und Kohlenhydraten zu bilden (Kubota K, Kageyama K, Shiro T und Okumura S (1971) Journal of General Applications in Microbiology, 17: 167-168; Kubota K, Kageyama K, Maeyashiki I, Yamada K und Okumura S (1972) Journal of General Applications in Microbiology 18: 365). An der Umsetzung von Glycin zu L-Serin ist hier das Enzym L-Serin-Hydroxymethyltransferase beteiligt (Kubota K und Yokozeki K (1989) Journal of Fermentation and Bioengeneering, 67(6):387-390). Die verwendeten Stämme weisen darüber hinaus einen verminderten L-Serin-Abbau auf, der auf eine Verringerung der Aktivität des Enzyms L-Serin-Dehydratase zurückzuführen ist (Kubota K, Kageyama K, Shiro T und Okumura S (1971) Journal of General Applications in Microbiology, 17: 167-168; Kubota K (1985) Agricultural Biological Chemistry, 49:7-12).

Weiterhin wird L-Serin fermentativ aus Methanol und Glycin unter Zuhilfenahme methylotropher Bakterien, wie z. B. *Hyphomicrobium* Stämmen, produziert (Izumi Y, Yoshida T, Miyazaki SS, Mitsunaga T, Ohshiro T, Shiamo M, Miyata A und Tanabe T (1993) Applied Microbiology and Biotechnology, 39: 427-432). In beiden Fällen muss die Aminosäure Glycin als Vorstufe für die Bildung der Aminosäure L-Serin eingesetzt werden.

Ferner sind coryneforme Bakterien bekannt, die L-Serin direkt aus Kohlenhydraten, ohne zusätzliche Beigabe weiterer Vorstufen produzieren können. Diese Stämme, die zu der Gattung *Corynebacterium glutamicum* gehören, weisen sich dadurch aus, dass sie z. B. resistent gegen die L-Serin-Analoga Serin-Hydroxamat und β-Chloroalanin sind und durch ungerichtete Mutagenese erhalten wurden (Yoshida H und Nakayama K (1974) Nihon-Nogei-Kagakukaishi 48: 201-208).

Darüber hinaus sind *Brevibacterium flavum* Stämme bekannt, die durch ungerichtete Mutagenese Defekte im L-Serin-Abbau aufweisen, eine erhöhte Aktivität der durch serA kodierten 3-Phosphoglycerat-Dehydrogenase besitzen, und die aus *Escherichia coli* stammenden Gene *serB* und *serC* überexprimieren (EP0931833A2). Das hierbei verwendete deregulierte *serA*-Gen wurde durch ungerichtete Mutagenese gewonnen und unterscheidet sich vom Wild Typ Gen nur durch einen einzigen Basenaustausch. Die Expression dieses Gens beinhaltet den Nachteil, dass es leicht zu einer Reveritierung und damit zur Zurückführung in den regulierten Zustand kommen kann.

Ein Nachteil bisher bekannter 3-Phosphoglycerat-Dehydrogenasen liegt in ihrer Feedback Inhibition durch L-Serin, wodurch beispielsweise die Produktivität der mikrobiellen Herstellung von L-Serin verringert wird. Die Region, die für diese Regulation durch L-Serin verantwortlich ist, ist der C-Terminus des Proteins. Aus WO 93/12235 ist eine DNA bekannt, die für eine 3-Phosphoglycerat-Dhydrogenase aus E. coli codiert, deren C-Terminus zu 25% verändert, komplett deletiert oder in den in einem bestimmten Bereich eine Insertion durchgeführt wurde, so dass eine geringere Inhibition durch L-Serin zu verzeichnen war. Diese 3-Phosphoglycerat-Dehydrogenase wies jedoch nur noch eine geringe Aktivität auf. Eine verbesserte L-Serinproduktion wurde mit der deregulierten 3-Phosphoglycerat-Dehydrogenase nicht nachgewiesen.

Die Wild Typ serA Sequenz ist allgemein bekannt und kann den dem Fachmann bekannten Datenbanken oder dem beigefügten Sequenzprotokoll gemäß SEQ ID No. 6 entnommen werden.

Die Schrift EP 0 943 687 A1 offenbart deregulierte Phosphoglycerat- Dehydrogenasen aus Corynebacterium, die Deletionen/ Substitutionen an der Position Glu325 tragen.

Die US-B1-6 180 373 beschreibt eine c- terminal deletierte PGDH aus E. coli, die bezüglich des Serinfeedbacks inhibitionsdesensitiert ist und die in Corynebakterien transfiziert wurde.

Es ist daher Aufgabe der Erfindung, Maßnahmen zur Verfügung zu stellen, mit denen die zuvor genannten Nachteile beseitigt werden können und die zu einer verbesserten Produktion von L-Serin oder davon ableitbaren Stoffwechselprodukten wie z. B. Tryptophan führen. Es ist somit Aufgabe der Erfindung Nukleinsäuren, codierend für eine 3-Phosphoglycerat-Dehydrogenase, bereitzustellen, die gegenüber natürlich vorkommenden 3-Phosphoglycerat-Dehydrogenasen eine verringerte Feedback Inhibition durch L- Serin unter Erhalt der Aktivität aufweist. In diesem Zusammenhang ist es weiterhin Aufgabe der Erfindung eine 3-Phosphoglycerat-Dehydro-genase sowie Mikroorganismen bereitzustellen, die gegenüber natürlich vorkommenden 3-Phosphoglycerat-Dehydrogenasen bzw. Mikroorganismen mit einer 3-Phosphoglycerat-Dehydrogenase, eine verringerte Feedback Inhibition durch L- Serin unter Erhalt der Aktivität aufweisen. Weiterhin ist es Aufgabe der Erfindung, ein verbessertes Verfahren zur mikrobiellen Herstellung von L- Serin bereitzustellen.

Ausgehend vom Oberbegriff des Anspruchs 1 wird die Aufgabe erfindungsgemäß gelöst, mit den im kennzeichnenden Teil des Anspruchs 1 angegebenen Merkmalen. Weiterhin wird die Aufgabe ausgehend vom Oberbegriff des Anspruchs 5 erfindungsgemäß gelöst, mit den im kennzeichnenden Teil des Anspruchs 5 angegebenen Merkmalen. Die Aufgabe wird außerdem ausgehend vom Oberbegriff des Anspruchs 6 erfindungsgemäß gelöst, mit den im kennzeichnenden Teil des Anspruchs 6 angegebenen Merkmalen. Die Aufgabe wird ebenso ausgehend vom Oberbegriff des Anspruchs 7 erfindungsgemäß gelöst, mit den im kennzeichnenden Teil des Anspruchs 7 angegebenen Merkmalen. Die Aufgabe wird weiterhin ausgehend vom Oberbegriff des Anspruchs 11 erfindungsgemäß gelöst, mit den im kennzeichnenden Teil des Anspruchs 11 angegebenen Merkmalen. Weiterhin wird die Aufgabe ausgehend vom Oberbegriff des Anspruchs 17 erfindungsgemäß gelöst, durch die im kennzeichnenden Teil des Anspruchs 18 angegebenen Merkmale.

Mit der erfindungsgemäßen Nukleinsäure sowie Polypeptid ist es nunmehr möglich, eine 3-Phosphoglycerat-Dehydrognase bereitzustellen, die gegenüber natürlich vorkommenden oder gentechnisch nicht veränderten Nukleinsäuren bzw. Enzymen keine bzw. eine verringerte L-Serin Feedback Inhibierung unter Erhalt der 3-Phosphoglycerat-Dehydrogenase Aktivität aufweist. Diese Eigenschaft wird im Folgenden unter der Bezeichnung "dereguliert" zusammengefasst. Weiterhin ist es möglich Mikroorganismen und Verfahren bereitzustellen, mit denen eine L-Serinproduktion mit gegenüber bisher bekannten mikrobiellen Verfahren höheren Ausbeuten möglich ist.

Vorteilhafte Weiterbildungen sind in den Unteransprüchen angegeben.

Gegenstand der Erfindung ist die Bereitstellung von Nukleinsäuren codierend für eine deregulierte 3-Phosphoglycerat-Dehydrogenase, im Folgenden mit PGD bezeichnet, enthaltend eine Gensequenz serA gemäß SEQ ID No 1. Die Nukleinsäure gemäß SEQ ID No 1, die für eine PGD mit einer Deletion von 197 Aminosäuren im C-Terminus codiert, hat sich als besonders vorteilhaft erwiesen.

Die erfindungsgemäßen Nukleinsäuren zeichnen sich dadurch aus, daß sie aus coryneformen Bakterien, bevorzugt der Gattung Corynebacterium oder Brevibacterium besonders bevorzugt aus Corynebacterium glutamicum, isoliert werden. Beispiele für in Stammkulturen hinterlegte Wild Typen coryneformer Bakterien sind, Corynebacterium glutamicum ATCC 13032 sowie Corynebacterium acetoglutamicum ATCC 15806 oder auch Brevibacterium flavum ATCC 14067. Beispiele für zur Herstellung von L-Serin geeignete Mutanten oder Produktionsstämme sind, Organismen aus der Gruppe Arthrobacter, Pseudomonas, Nocardia, Methylobacterium, Hyphomycrobium, Alcaligenes oder Klebsiella. Die vorliegende Erfindung wird durch die Angabe der zuvor genannten Bakterienstämme näher charakterisiert, die jedoch nicht limitierend wirkt.

Unter einer Nukleinsäure oder einem Nukleinsäurefragment ist erfindungsgemäß ein Polymer aus RNA oder DNA zu verstehen, das einzel- oder doppelsträngig sein kann und optional natürliche, chemisch synthetisierte, modifizierte oder artifizielle Nukleotide enthalten kann. Der Begriff DNA-Polymer schließt hierbei auch genomische DNA, cDNA oder Mischungen davon ein.

Erfindungsgemäß sind auch die den codierenden Bereichen (Strukturgenen) vorausgehenden (5'-oder upstream) und/oder nachfolgenden (3'-oder downstream) Sequenzbereiche eingeschlossen. Insbesondere sind hierin Sequenzbereiche mit regulatorischer Funktion inbegriffen. Sie können die Transkription, die RNA-Stabilität oder die RNA Prozessierung sowie die Translation beeinflussen. Beispiele für regulatorische Sequenzen sind u.a. Promotoren, Enhancer, Operatoren, Terminatoren oder Translationsverstärker.

Gegenstand der Erfindung ist ferner eine Genstruktur enthaltend wenigstens eine der zuvor beschriebenen Nukleotidsequenzen codierend für eine deregulierte PDG sowie mit diesen operativ verknüpfte regulatorische Sequenzen, welche die Expression der codierenden Sequenzen in der Wirtszelle steuern.

Darüber hinaus betrifft die vorliegende Erfindung einen Vektor enthaltend eine Nukleotidsequenz der zuvor beschriebenen Art codierend für eine deregulierte PDG, mit diesen operativ verknüpfte regulative Nukleotidsequenzen sowie zusätzliche Nukleotidsequenzen zur Selektion transformierter Wirtszellen, für die Replikation innerhalb der Wirtszelle oder zur Integration in das entsprechende Wirtszell-Genom. Ferner kann der erfindungsgemäße Vektor eine Genstruktur der vorgenannten Art enthalten.

Als Vektoren eignen sich solche, die in coryneformen Bakterien repliziert werden wie z. B. pZ1 (Menkel E, Thierbach G, Eggeling L, Sahm H., 1989, Appl Environ Microbiol 55(3): 684-688), pEKEx2 (Eikmanns et al., Gene 102: 93-98 (1991) , pVWEx oder pXMJ19. Andere Plasmidvektoren können in gleicher Weise verwendet werden. Diese Aufzählung ist für die vorliegende Erfindung jedoch nicht limitierend.

Unter Ausnutzung der erfindungsgemäßen Nukleinsäuresequenzen können entsprechende Sonden oder auch Primer synthetisiert und dazu verwendet werden, beispielsweise mit Hilfe der PCR-Technik analoge Gene aus anderen Mikroorganismen, bevorzugt coryneformen Bakterien zu amplifizieren und isolieren.

Gegenstand der vorliegenden Erfindung ist ferner eine deregulierte PGD oder ein Teil davon, kodiert durch eine erfindungsgemäße Nukleinsäuresequenz gemäß SEQ ID No 1 oder deren Variationen der zuvor beschriebenen Art. Die vorliegende Erfindung betrifft ebenso eine deregulierte PGD mit einer Aminosäuresequenz gemäß der SEQ ID No 7 oder einer modifizierten Form dieser Polypeptidsequenzen oder Isoformen davon oder Mischungen daraus. Als besonders geeignet hat sich eine 3-Phosphoglycerat- Dehydrogenase mit einer Aminosäuresequenz gemäß SEQ ID No 7 erwiesen.

Gegenstand der vorliegenden Erfindung sind Polypeptide mit der Funktion einer deregulierten PGD, die in ihrer Aminosäuresequenz derart verändert sind, dass sie gegenüber regulatorisch wirkenden Verbindungen, beispielsweise die sie in ihrer Aktivität regulierenden Stoffwechsel-Endprodukte (L-Serin) desensitiv sind (feedback-desensitiv).

Die erfindungsgemäßen Polypeptide zeichnen sich dadurch aus, daß sie aus coryneformen Bakterien, bevorzugt der Gattung Corynebacterium oder Brevibacterium, besonders bevorzugt der Art Corynebacterium glutamicum oder Brevibacterium besonders bevorzugt aus Corynebacterium glutamicum stammen. Beispiele für in Stammkulturen hinterlegte Wild Typen coryneformer Bakterien sind Corynebacterium glutamicum ATCC 13032, sowie Corynebacterium acetoglutamicum ATCC 15806 oder auch Brevibacterium flavum ATCC 14067. Beispiele für zur Herstellung von L-Serin geeignete Mutanten oder Produktionsstämme sind Organismen aus der Gruppe Arthrobacter, Pseudomonas, Nocardia, Methylobacterium, Hyphomycrobium, Alcaligenes oder Klebsiella. Die vorliegende Erfindung wird durch die Angabe der zuvor genannten Bakterienstämme näher charakterisiert, die jedoch nicht limitierend wirkt.

Gegenstand der vorliegenden Erfindung ist ferner die Übertragung wenigstens einer der erfindungsgemäßen Nukleinsäuresequenzen in ein Wirtssystem. Dies schließt auch die Übertragung eines erfindungsgemäßen Genkonstrukts oder Vektors in ein Wirtssystem ein. Diese Übertragung von DNA in eine Wirtszelle erfolgt nach gentechnischen Methoden. Als bevorzugtes Verfahren sei hier die Transformation und besonders bevorzugt die Übertragung von DNA durch Elektroporation genannt.

Als besonders geeignet hat sich ein homologes Wirtssystem erwiesen. Unter einem homologen Wirtssystem sind Mikroorganismen zu verstehen, die alle einer verwandten Familie angehören. Erfindungsgemäß sind hierunter coryneforme Bakterien zu verstehen, in die die erfindungsgemäß aus coryneformen Bakterien isolierten Nukleinsäuren eingebracht werden. Ein aus einer erfolgreich durchgeführten Nukleinsäureübertragung resultierender transformierter Mikroorganismus unterscheidet sich somit von dem entsprechend nicht transformierten Mikroorganismus dadurch, dass er zusätzliche Nukleinsäuren der erfindungsgemäßen Art enthält und entsprechend zur Ausprägung bringen kann. Stellvertretend für ein geeignetes homologes Wirtssystem sei das Bakterium Corynebacterium glutamicum und bevorzugt der Stamm ATCC 13032 genannt. Als Kulturmedium ist je nach Anforderungen ein Komplexmedium wie z . B. LB Medium (T. Maniatis, E.F. Fritsch und J. Sambrook, Molecular Clonin : A Laboratory Manual, Cold Spring Harbor Laboratory, Cold Spring Harbor, NY 1989)) oder auch ein Mineralsalzmedium, wie z. B. CGXII-Medium (Keilhauer, C. et al 1993, J. Bacteriol., 175:5593-5603) geeignet. Nach entsprechender Kultivierung kann die Bakteriensuspension geerntet und zur weiteren Untersuchung, beispielsweise zur Transformation oder zur Isolierung von Nukleinsäuren nach gängigen Methoden eingesetzt werden. Diese Vorgehensweise kann analog auch auf andere coryneforme Bakterienstämme angewendet werden. Dabei werden als Wirtssysteme Bakterien der Gattung Corynebacterium oder Brevibacterium bevorzugt. Innerhalb der Gattung Corynebacterium wird besonders die Art Corynebacterium glutamicum und innerhalb der Gattung Brevibacterium besonders die Art Brevibacterium flavum bevorzugt. Zu den Vertretern dieser Gattungen zählen zum einen Stämme, die in ihren Eigenschaften als Wild Typ charakterisiert sind. Hier sind beispielsweise Corynebacterium glutamicum ATCC 13032, Corynebacterium glutamicum ATCC 14752, Corynebacterium acetoglutamicum ATCC 15806, Corynebacterium acetoglutamicum ATCC 15806, Corynebacterium melassecola ATCC 17965, Corynebacterium thermoaminogenes FERM BP-1539, Brevibacterium flavum ATCC 14067, Brevibacterium lactofermentum ATCC 13869 und Brevibacterium divaricatum ATCC 14020 zu nennen.

Darüber hinaus schließt die vorliegende Erfindung auch Bakterienstämme als Wirtssystem ein, die sich als L-Serin produzierende Mutanten oder Aminosäureproduktionsstämme auszeichnen. Diese können z. B. ausgehend von Wildtypstämmen durch klassische (chemische oder physikalische) oder gentechnische Methoden hergestellt werden. Beispiele für erfindungsgemäß geeignete Stämme sind u. a. Corynebacterium glutamicum ATCC 21586, Corynebacterium glutamicum KY 10150, Corynebacterium glutamicum ATCC 13032ΔpanBC und Brevibacterium ketoglutamicum ATCC 21222. Ferner sind erfindungsgemäß auch diejenigen Produktionsstämme geeignet, die dem Fachmann aus mikrobiellen Herstellungsverfahren bekannt sind, wie z. B. Enterobacterien, Bacillaceen oder Hefe-Arten. Die vorliegende Erfindung wird durch die ausgewählten Beispiele an Mikroorganismen näher charakterisiert, jedoch nicht limitiert.

Die vorliegende Erfindung betrifft ferner einen genetisch veränderten Mikroorganismus enthaltend in replizierbarer Form eine erfindungsgemäße Nukleinsäure der zuvor beschriebenen Art, welche im Vergleich zu dem entsprechend nicht genetisch veränderten Mikroorganismus verstärkt exprimiert wird und/oder deren Kopienzahl erhöht ist.

Ebenso umfasst die vorliegende Erfindung einen genetisch veränderten Mikroorganismus enthaltend in replizierbarer Form eine Genstruktur oder einen Vektor der zuvor beschriebenen Art.

Gegenstand der vorliegenden Erfindung ist darüber hinaus auch ein genetisch veränderter Mikroorganismus enthaltend ein erfindungsgemäßes Polypeptid mit der Funktion einer deregulierten PGD der zuvor beschriebenen Art, welches eine im Vergleich zu dem entsprechend nicht genetisch veränderten Mikroorganismus eine verringerte bzw. keine feedback Inhibierung durch L-Serin unter Erhalt der PGD-Aktivität aufweist. Ein erfindungsgemäß genetisch veränderter Mikroorganismus zeichnet sich ferner dadurch aus, daß er ein coryneformes Bakterium, bevorzugt der Gattung Corynebacterium oder Brevibacterium, besonders bevorzugt der Spezies Corynebacterium glutamicum oder Brevibacterium flavum ist.

Prinzipiell können Gene durch an sich bekannte Methoden, wie beispielsweise die Polymerase-Ketten-Reaktion (PCR) mit Hilfe von kurzen, synthetischen Nukleotidsequenzen (Primern) amplifiziert und anschließend isoliert werden. Die Herstellung der verwendeten Primer erfolgt im Allgemeinen anhand bekannter Gensequenzen aufgrund bestehender Homologien in konservierten Bereichen der Gene und/oder unter Berücksichtigung des GC-Gehalts der DNA des zu untersuchenden Mikroorganismus.

Eine weitere Vorgehensweise zur Isolierung von codierenden Nukleotidsequenzen ist die Komplementation von sogenannten Defekt-Mutanten des zu untersuchenden Organismusses, die zumindest phänotypisch einen Funktionsverlust in der Aktivität des zu untersuchenden Gens oder entsprechenden Proteins aufweisen. Unter einer Komplementation ist die Aufhebung des Gendefektes der Mutante und weitgehende Wiederherstellung des ursprünglichen Erscheinungsbildes vor der Mutagenese zu verstehen, die durch die Einbringung funktioneller Gene oder Genfragmente aus dem zu untersuchenden Mikroorganismus erreicht wird.

Ein klassisches Mutagenese-Verfahren zur Herstellung von Defektmutanten ist beispielsweise die Behandlung der Bakterienzellen mit Chemikalien wie z. B. N-Methyl-N-Nitro-N-Nitrosoguanidin oder UV-Bestrahlung. Derartige Verfahren zur Mutationsauslösung sind allgemein bekannt und können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA, 1981) nachgelesen werden.

Die vorliegende Erfindung betrifft darüber hinaus ein Verfahren zur mikrobiellen Herstellung von L-Serin, wobei wenigstens eine der erfindungsgemäßen Nukleinsäuren, isoliert aus einem coryneformen Bakterium, in einen Wirtsorganismus übertragen und dort exprimiert werden, wobei die Genexpression und/oder die Aktivität des entsprechend kodierten Polypeptids gegenüber dem entsprechend nicht genetisch veränderten Mikroorganismus erhöht ist, dieser genetisch veränderte Mikroorganismus zur mikrobiellen Herstellung von L-Serin eingesetzt wird und das entsprechend gebildete L-Serin aus dem Kulturmedium isoliert wird.

Zur Erzielung einer erhöhten Genexpression (Überexpression) kann die Kopienzahl der entsprechenden Gene erhöht werden. Ferner kann die Promotor- und/oder Regulationsregion und/oder die Ribosomenbindungsstelle, die sich stromaufwärts des Strukturgens befindet, entsprechend so verändert werden, dass die Expression mit erhöhter Rate erfolgt. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Serin Produktion zu steigern. Durch Maßnahmen zur Verlängerung der Lebensdauer der mRNA wird ebenfalls die Expression verbessert. Die Gene oder Genkonstrukte können entweder in Plasmiden mit unterschiedlicher Kopienzahl vorliegen oder im Chromosom integriert und amplifiziert sein. Weiterhin kann auch die Aktivität des Enzyms selbst erhöht sein oder durch die Verhinderung des Abbaus des Enzymproteins verstärkt werden. Alternativ kann ferner eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (BioRechnology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Die erfindungsgemäß hergestellten genetisch veränderten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) zum Zwecke der Produktion von L-Serin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlenhydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzu gegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe z. B. Antibiotika hinzugefügt werden. Um aerobe Bedingungen aufrechtzuerhalten werden Sauerstoff oder sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird so lange fortgesetzt, bis sich ein Maximum an L-Serin gebildet hat. Dieses Ziel wird normalerweise innerhalb von 10 Stunden bis 160 Stunden erreicht.

Die Analyse der L-Serin-Bildung kann durch Anionenaustauschchromatographie mit anschließender Ninhydrin Derivatisierung erfolgen so wie bei Spackman et al. (Analytical Chemistry, 30, (1958), 1190) beschrieben, oder sie kann durch reversed Phase HPLC erfolgen so wie bei Lindroth et al. (Analytical Chemistry (1979) 51: 1167-1174) beschrieben.

Die Mikroorganismen, die Gegenstand der vorliegenden Erfindung sind, können L-Serin aus Glucose, Saccharose, Lactose, Mannose, Fructose, Maltose, Melasse, Stärke, Cellulose oder aus Glycerin und Ethanol herstellen. Es kann sich um die zuvor bereits näher beschriebenen Vertreter coryneformer Bakterien handeln. Eine Auswahl an Ergebnissen der Fermentation ist in Tabelle 6 dargestellt. Hierbei zeichnen sich die erfindungsgemäß genetisch veränderten Mikroorganismen durch eine wesentlich verbesserte L-Serin-Produktion gegenüber den entsprechend nicht transformierten Mikroorganismen (Wild Typen) oder den Mikroorganismen aus, die lediglich den Vektor ohne Gen-Insert enthalten. In einer besonderen Ausführungsvariante der vorliegenden Erfindung ist gezeigt, dass die Überexpression des homologen C-terminal verkürzten serA-Gens in C. glutamicum ATCC 13032DpanBCpZ1serAΔ197 zu einer wenigstens 40%igen Steigerung der L-Serin Akkumulation im Medium im Vergleich zu den Kontrollstämmen führt (Tab. 6). Durch die gemeinsame Überexpression weiterer Gene, die positiv auf den L-Serinbiosyntheseweg wirken, ist eine noch weitere Steigerung der L-Serin-Produktion zu erwarten.

Unter Aminosäure-Produktionsstämmen sind im Sinne der vorliegenden Erfindung Corynebacterium glutamicum-Stämme oder homologe Mikroorganismen zu verstehen, die durch klassische und/oder molekulargenetische Methoden derart verändert sind, dass ihr Stoffwechselfluss verstärkt in die Richtung der Biosynthese von Aminosäuren oder deren Abkömmlingen verläuft (metabolic engineering). Beispielsweise sind bei diesen Aminosäure-Produktionsstämmen ein oder mehrere Gen(e) und/oder die korrespondierenden Enzyme, die an entscheidenden und entsprechend komplex regulierten Schlüsselpositionen des Stoffwechselweges (Flaschenhals) stehen in ihrer Regulation verändert oder sogar dereguliert. Die vorliegende Erfindung umfasst hierbei sämtliche bereits bekannte Aminosäure-Produktionsstämme, bevorzugt der Gattung Corynebacterium oder homologer Organismen. Ferner sind erfindungsgemäß auch diejenigen Produktionsstämme umfaßt, die der Fachmann in Analogie zu Erkenntnissen aus anderen Mikroorganismen, beispielsweise Enterobacterien, Bacillaceen oder Hefe-Arten nach gängigen Methoden herstellen kann.

Die Figuren zeigen beispielhaft verwendete Plasmide sowie einen Vergleich der Primärstruktur der PGD und mittels PCR konstruierter Allele von *serA*.

Es zeigt:

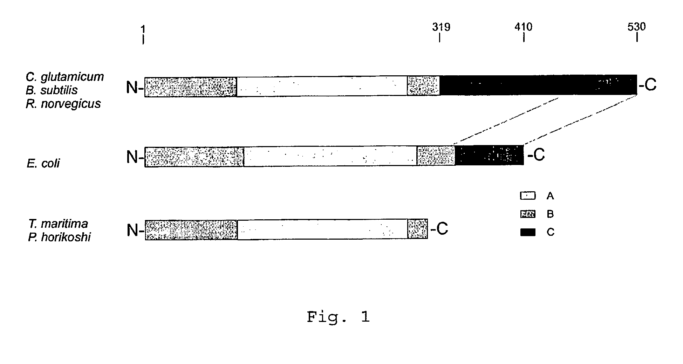

Fig. 1: Vergleich der Primärstruktur der 3-Phosphoglycerat-Dehydrogenase (PGD) aus verschiedenen Organismen; Skalierung entspricht der Anzahl an Aminosäuren der corynebakteriellen PGD; N = Aminoterminus; C = Carboxyterminus; der mit einer hell grauen Fläche markierte Bereich A stellt die Nukleotid-Bindungsstelle dar; der mit einer dunkel grauen Fläche markierte Bereich B stellt die Substrat-Bindungsstelle dar; der schwarz markierte Bereich C stellt die Inhibitor-Bindungsstelle dar.
   Darüber hinaus gibt es zwei weitere Gruppen von 3-Phosphoglycerat-Dehydrogenasen, die exemplarisch durch E. *coli* (Tobey K.L. und Grant G.A., 1986, J. Biol. Chem., 261: 12179-12183) bzw. *Thermotoga maritima* (GenBank-Accession-Nummer AE000512) vertreten sind. Hierbei ist das Protein des hyperthermophilen Bakteriums *T. maritima* mit einer Länge von 327 Aminosäuren am kürzesten, während die 3-Phosphoglycerat-Dehydrogenase aus *E. coli* mit 410 Aminosäuren eine intermediäre Länge aufweist.
Fig. 2: Übersicht über die mittels PCR konstruierten Allele von *serA,* die für die deregulierte, C-terminal verkürzte PGD codieren. Gezeigt ist der serA-Genbereich des Wild Typs (oben) und die erfindungsgemäßen Deletionskonstrukte. Die hell, dunkel und schwarz markierten Bereiche entsprechen der Definition wie in Fig. 1.
Fig. 3: Plasmidvektor pZ1*serA*
Fig. 4: Plasmidvektor pZ1*serA*Δ79
Fig. 5: Plasmidvektor pZ1*serA*Δ188
Fig. 6: Plasmidvektor pZ1*serA*Δ197
Fig. 7: Plasmidvektor pZ1*serA*Δ205
Fig. 8: Plasmidvektor pZ1*serA*Δ211

### Ausführungsbeispiele:

### 1. Gezielte Deregulation der 3-Phosphoglycerat-Dehydrogenase aus C. glutamicum

### a) Computergestützter Aminosäuresequenz-Vergleich der

### 3-Phosphoglycerat-Dehydrogenase aus Corynebacterium glutamicum mit 3-Phosphoglycerat-Dehydrogenasen anderer Organismen

Es wurde zunächst eine Strategie zur Konstruktion einer deregulierten 3-Phosphoglycerat-Dehydrogenase entwickelt. Es wurde die Sequenz des serA-Gens, das für die 3-Phosphoglycerat-Dehydrogenase von *C. glutamicum* kodiert, aus der Patent-Datenbank verwendet (Nakagawa,S., Mizoguchi,H., Ando,S., Hayashi,M., Ochiai,K., Yokoi,H., Tateishi,N., Senoh,A., Ikeda,M. and Ozaki,A. Patent: EP 1108790-A 7064 20-JUN-2001; KYOWA HAKKO KOGYO CO., LTD. (JP); Pompejus,M., Kroeger,B., Schroeder,H., Zelder,O. and Haberhauer,G.Patent: WO 0100843-A 167 04-JAN-2001; BASF AKTIENGESELLSCHAFT (DE)). Die vom serA-Gen (SEQ-ID-No. 12) von *Corynebacterium glutamicum* abgeleitete Polypeptidkette wurde dann mit entsprechenden 3-Phosphoglycerat-Dehydrogenasen aus der Datenbank (GenBank) verglichen. Es zeigte sich, dass die 3-Phosphoglycerat-Dehydrogenase aus *C. glutamicum* wie die aus *Mycobacterium tuberculosis* (GenBank-Accession-Nummer AL123456) und einigen anderen Bakterien wie Ba*cillus subtilis* (Sorokin,A., Azevedo,V., Zumstein,E., Galleron,N., Ehrlich,S.D. und Serror,P. Microbiology 142 (Pt 8), 2005-2016 (1996)) und *Aquifex aeolicus* (GenBank-Accession-Nummer AE000657) mit 530 Aminosäuren ausserordentlich lang ist. Zu dieser Gruppe von Enzymen zählen auch die 3-Phosphoglycerat-Dehydrogenasen aus Tieren wie Ratte (Achouri Y., Rider M.H., Van Schaftingen E. und Robbi M., 1997, Biochem. J., 323:365-370) und Mensch (Cho HM, Jun DY, Bae MA, Ahn JD, Kim YH., 2000, Gene 245(1) :193-201) sowie Pflanzen (z. B. *Arabidopsis thaliana*; Ho CL, Saito K., 2001, Amino Acids. 20(3):243-59). Die Analyse der Röntgenstruktur des E. coli-Enzyms ergab, dass es aus drei funktionellen Domänen besteht: einer Nukleotidbindedomäne (Aminosäure 108 bis 294) für die Bindung von NAD/H, einer zweigeteilten Substratbindedomäne (Aminosäure 7-107 und 295-336), an der das 3-Phosphoglycerat bindet, sowie einer C-termi-nalen regulatorischen Domäne (Aminosäure 337-410), die für die allosterische Bindung des L-Serin verantwortlich ist (Schuller DJ, Grant GA, Banaszak LJ., 1995, Nature Struct. Biol. Vol 2 1:69-76). Der Aminosäuresequenzvergleich der drei 3-Phosphoglycerat-Dehydro-genase-Typen ergab, dass sie sich im Wesentlichen in der Länge der C-terminalen regulatorischen Domäne unterscheiden (Abb. 1).

Eine Clusteranalyse der 3-Phosphoglycerat-Dehydrogenasen, die aus vollständig sequenzierten Genomen bekannt sind, ergab, dass trotz der Unterschiede im C-Terminus alle diese Proteine zu einer Familie von Orthologen zählen, d. h. sie besitzen einen gemeinsamen evolutiven Ursprung, haben sich aber in den verschiedenen Spezies unterschiedlich entwickelt.

### b) Konstruktion von Allelen des serA-Gens von C. glutamicum mittels PCR die für C-terminal verkürzte 3-Phosphoglycerat-Dehydrogenase Proteine codieren

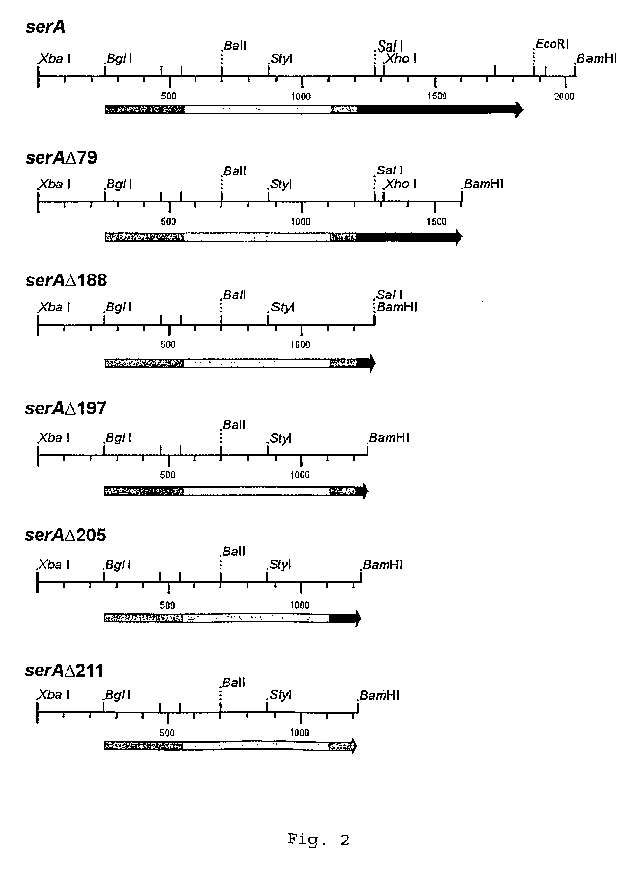

Es wurden fünf verschiedene Muteine der 3-Phosphoglycerat-Dehydrogenase von *C. glutamicum* erzeugt, die am C-Terminus unterschiedlich lange Deletionen aufwiesen (Abb. 2). Die Konstruktion der Deletionsmutanten erfolgte ebenso wie die Isolierung des Wild Typ *serA-*Gens mittels PCR. Hierzu wurde ein PCR-Primer (serA-f: 5'-TCTAGAGCCGGAGACGTGAATAAAAT-3') erzeugt, der homolog zu einer Region 240 bp vor dem Start-Codon des Gens war, um so den gesamten Promotorbereich zu erfassen. Dieser Primer wurde für alle Konstrukte gleichermaßen verwendet und trägt am 3'-Ende eine Schnittstelle für das Restriktionsenzym *Xba*I. Zur Amplifikation des vollständigen serA-Gens wurde ein zweiter, revers-komplementärer Primer ausgewählt, der 199 bp hinter dem Stop-Codon lag und eine BamHI-Restriktionsschnittstelle trägt (serA-r: 5'-GGATCCGACTGGTGAGGGTCAAGTCC-3'). Das erwartete PCR-Produkt hat eine Länge von 2040 bp. Zur Erzeugung der Deletionen wurden revers-komplementäre Primer ausgewählt, die im Genbereich liegen, und alle ebenfalls eine Schnittstelle für *Bam*HI tragen. Der Primer *serA*Δ211-r (5'-GGATCCTTAACCGGAAACGTTCACAGC3') liegt 956 bp hinter dem Start-Codon, so dass ein 1196 bp langes PCR-Produkt entsteht. Hierdurch werden die letzten 211 Aminosäuren der 3-Phosphoglycerat-Dehydrogenase abgeschnitten. Die Deletion liegt etwa im Bereich des vermutlichen Übergangs von Substratbinde- zu regulatorischer Domäne (vergl. Abb. 1 und Abb. 2). Der Primer *serA*Δ205-r (5'-GGATCCTTACTCTTCGCCCACGCGACC3') liegt 974 bp hinter dem Start-Codon und das zu erwartende PCR-Produkt hat eine Länge von 1214 bp. Die C-terminale Deletion beträgt in diesem Fall 205 Aminosäuren und das Protein endet hinter der Aminosäure Glutamat an Position 325. Der ungerichtet erzeugte Austausch dieser Aminosäure zu Lysin führt in *C. glutamicum* zu einer Deregulation der 3-Phosphoglycerat-Dehydrogenase (EP 0 931 833). Beide Deletionen liegen in einem Bereich, in dem auch die Deletion (Δ209 Aminosäuren) des Proteins aus Ratte erzeugt wurde (Achouri Y., Rider M.H., Van Schaftingen E. und Robbi M., 1997, Biochem. J., 323:365-370). Die beiden Primer *serA*Δ197-r (5'-GGATCCTTAAGCCAGATCCATCCACACAG3') und *serA*Δ188-r (5'-GGATCCTTACTTGCCAGCAAGAAGACC3') liegen 998 bp bzw. 1025 bp hinter dem ATG und befinden sich stromaufwärts vom Übergang Substratbindedomäne zu regulatorischer Domäne in E. *coli.* Die nach PCR zu erwartenden DNA-Fragmente erzeugen Polypeptidketten die entsprechend um 197 bzw. 188 Aminosäuren kürzer sind als die vollständige 3-Phosphoglycerat-Dehydrogenase. Die kürzeste Deletion wird durch Primer *serA*Δ79-r (5'-GGATCCTTAATCCAGGCCACGGCCATT3') erzeugt und schneidet den Bereich von 79 Aminosäuren ab, der die größte Ähnlichkeit zur regulatorischen Domäne von E. *coli* aufweist (Abb.2). Zusätzlich wurde in allen reverskomplementären Primern, die zu einem verkürzten Protein führen sollen hinter der Schnittstelle das Stop-Codon TAA eingefügt.

Die PCR-Reaktion wurde in 30 Zyklen in Gegenwart von 200 µM Deoxynukleotid-triphosphaten (dATP, dCTP, dGTP, dTTP), je 1 µM des entsprechenden Oligonukleotids, 100 ng chromosomaler DNA von *Corynebacterium glutamicum* ATCC13032, 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 60 Sekunden, 50°C für 90 Sekunden und 72°C für 2 Minuten.

Nach der PCR-Reaktion wurden die erhaltenen DNA-Fragmente mit dem QIAExII Gelextraktionskit (Qiagen) nach Angaben des Herstellers aus einem 0,8 %igen Agarose-Gel isoliert, blunt-end mit Hilfe des Sure Clone-Kits (Amersham Pharmacia Biotech) in die SmaI-Schnittstelle des Vektors pUC18 kloniert. Die Plasmide wurden durch Restriktionskartierung auf Richtigkeit überprüft. Diese Klonierung erfolgte in dem Escherichia *coli* Stamm DH5αmcr (Grant et al., Proceedings of the National Academy of Sciences of the United States of America USA (1990) 87: 4645-4649).

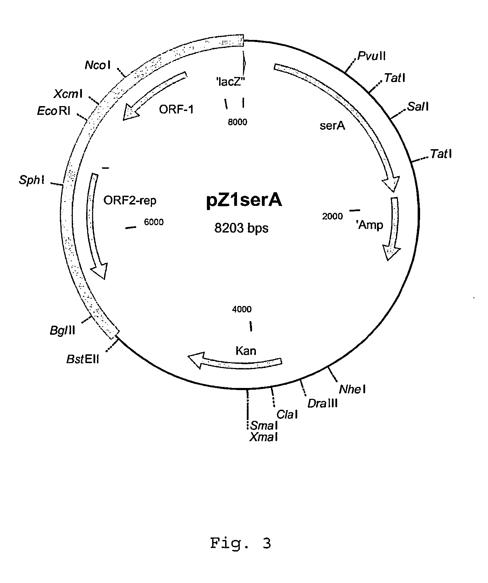

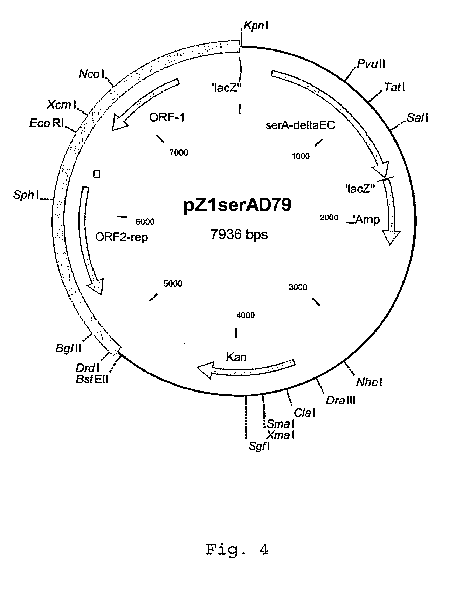

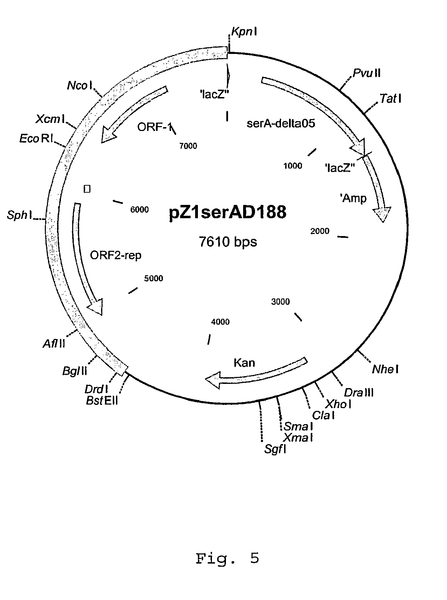

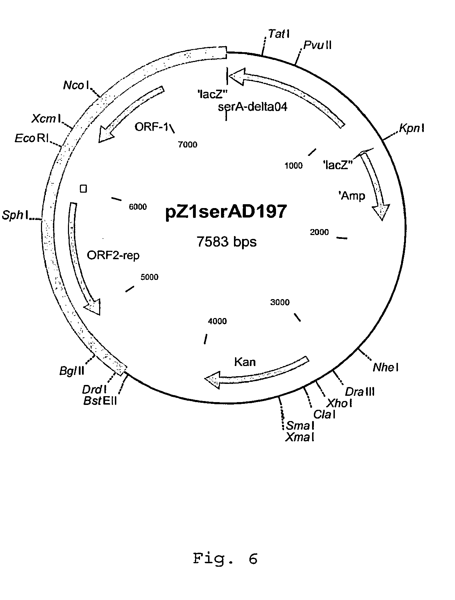

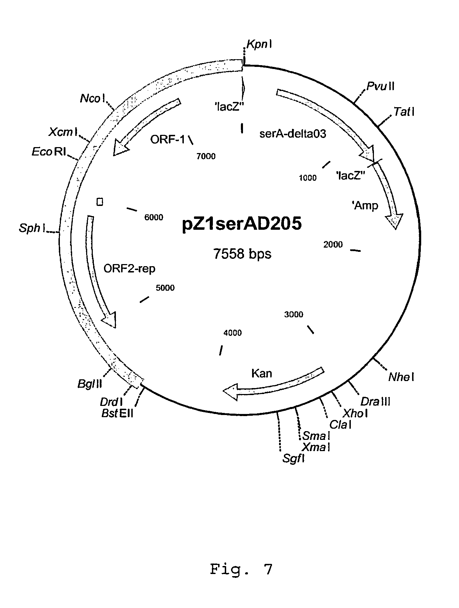

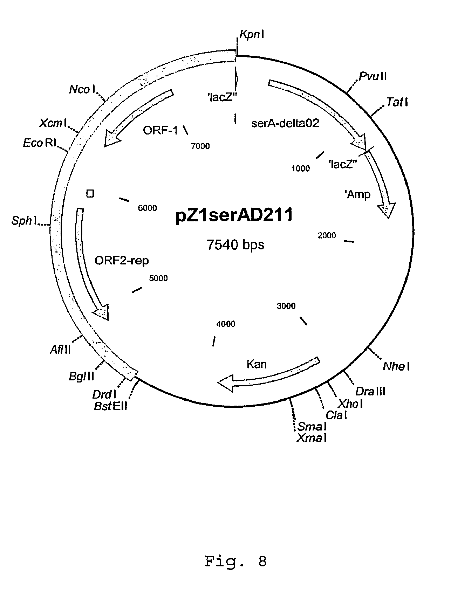

Anschließend wurde das serA-Gen und die serA-Deletionskonstrukte in den *E. coli*/*C*. *glutamicum* Pendelvektor pZ1 (Menkel E, Thierbach G, Eggeling L, Sahm H., 1989, Appl Environ Microbiol 55(3): 684-688) kloniert. Der Vektor vermittelt eine Kanamycin-Resistenz. Hierzu wurden die Inserts der Deletionskonstrukte jeweils mit den Restriktionsenzymen *Eco*RI und *Bam*HI aus dem pUC18-Vektor ausgeschnitten. Die überhängenden DNA-Enden wurden mittels Klenow-Behandlung aufgefüllt und die Fragmente wurden blunt-end in den ScaI-geschnittenen Vektor pZ1 ligiert. Das Wild Typ serA-Gen wurde nach EcoRI-Restriktion ebenfalls Klenow behandelt und blunt-end in den *Sca*I-geschnittenen Vektor pZ1 ligiert. Die so erhaltenen Konstrukte wurden pZ1*serA* (Abb. 3), pZ1*serA*Δ79 (Abb. 4), pZ1*serA*Δ188 (Abb. 5), pZ1*serA*Δ197 (Abb. 6), pZ1*serA*Δ205 (Abb. 7) und pZ1*serA*Δ211 (Abb. 8) genannt.

### 2. Überexpression des Wild Typ serA-Gens sowie der verkürzten serA-Allele in C. glutamicum

Die Plasmide pZ1*serA*, pZ1*serA*Δ79, pZ1*serA*Δ188 pZ1*serA*Δ197 pZ1*serA*Δ205 und pZ1*serA*Δ211 wurden durch Elektroporation einzeln in *C. glutamicum* eingebracht. Als Kontrolle wurde das Leerplasmid pZ1 ebenfalls nach *C. glutamicum* ATCC 13032 elektroporiert. Die so erhaltenen Stämme 13032pZ1, 13032pZ1*serA*, 13032pZ1*serA*Δ79, 13032pZ1*serA*Δ188, 13032pZ1*serA*Δ197, 13032pZ1*serA*Δ205 und 13032pZ1*serA*Δ211 wurden dann auf Überexpression der 3-Phosphoglycerat-Dehydrogenase mittels 3-Phosphoglycerat-Dehydrogenase-Enzymtest analysiert. Hierzu wurden die sechs Stämme in Komplexmedium (CgIII = 2,5 g NaCl, 10 g Bacto-Peptone, 10 g Bacto-Yeast Extract, pH 7,4 mit 2 % Glukose) gezüchtet, und das Minimalmedium CGXII jeweils aus den vorkulturen getrennt beimpft. Das Medium war identisch mit dem bei Keilhauer et al. beschriebenen Medium CGXII (Journal of Bacteriology (1993) 175: 5593-5603), enthielt aber zusätzlich 25 µg/mL Kanamycin. Die Zusammensetzung des von Keilhauer et al. beschriebenen Mediums ist in Tabelle 1 dargestellt.

**Tabelle 1: Zusammensetzung des Mediums CGXII**

| Komponente | Konzentration |
|---|---|
| (NH₄)₂SO₄ | 20 g/L |
| Harnstoff | 5 g/L |
| KH₂PO₄ | 1 g/L |
| K₂HPO₄ | 1 g/L |
| MgSO₄ x 7 H₂O | 0,25 g/L |
| 3-Morpholinopropansulfonsäure | 42 g/L |
| CaCl₂ | 10 mg/L |
| FeSO₄ x 7 H₂O | 10 mg/L |
| MnSO₄ x H₂O | 10 mg/L |
| ZnSO₄ x 7H₂O | 1 mg/L |
| CuSO₄ | 0,2 mg/L |
| NiCl₂ x 6 H₂O | 0,02 mg/L |
| Biotin | 0,2 mg/L |
| Glukose | 40 g/L |
| Protokatechusäure | 30 mg/L |

Die Zellen wurden in der exponentiellen Wachstumsphase bei OD₆₀₀ von 5 bis 8 geerntet und zweimal in 100 mM Tris-HCl, pH 7,5 gewaschen. Die Zellpellets wurden anschliessend bis zum Aufschluss bei -20°C eingefroren. Die eingefrorenen Zellpellets wurden dann auf Eis aufgetaut und mit 2 ml kaltem 100 mM Tris-HCl pH 7,5/10 % Glycerin resuspendiert und in einem Brenson Sonifier 10 min aufgeschlossen. Anschließend wurden die Zelltrümmer mittels Zentrifugation bei 13000 rpm und 4°C in einer Sigma -202 MK zentrifuge abgetrennt. Die so erhaltenen Überstände wurden als Rohextrakte zunächst über eine PD-10 Säule nach Angaben des Herstellers (Amersham Pharmacia Biotech) entsalzt und dann sofort in die Enzymmessung eingesetzt. Der Enzymtest beruht auf dem photometrischen Nachweis der Bildung von NADH in der Reaktion 3-Phosphoglycerat und NAD zu Phosphohydroxypyruvat zu NADH. Der Testansatz ist in Tabelle 2 dargestellt:

**Tabelle 2: Komponenten des Testansatzes zur Bestimmung der 3-Phosphoglycerat-Dehydrogenase Aktivität**

| | Stammlösung | Endkonzentration |
|---|---|---|
| Tris-HCl; pH 8.8 | 500 mM | 100 mM |
| Dithiothreit | 100 mM | 1 mM |
| EDTA | 500 mM | 5 mM |
| Hydrazin | 250 mM | 10 mM |
| NAD | 20 mg/ml | 2 mg/ml |
| RE | ca. 2 mg/ml | ca. 200 µg Protein |
| 3-Phosphoglycerat | 150 mM | 15 mM |

Mit diesem Testansatz konnte für die 3-Phosphoglycerat-Dehydrogenase des Wild Typs eine spezifische Aktivität von ca. 150 mU/mg Protein bestimmt werden. Es zeigte sich, dass die Überexpression des vollständigen *serA-*Gens eine etwa 16-fache Steigerung der spezifischen 3-Phosphoglycerat-Dehydrogenase-Aktivität ergibt. Das Konstrukt *serA*Δ197 ermöglichte eine 10-fache Überexpression gegenüber dem Wild Typ-Protein. Die Konstrukte *serA*Δ188 und *serA*Δ205 lassen sich 3 bis 3,4-fach überexprimieren, wohingegen für die Konstrukte *serA*Δ205 und *serA*Δ79 nur eine 1,2 bis 1,5-fache Überexpression möglich war. Damit ist gezeigt, dass das durch Deletion der C-terminalen 197 Aminosäuren der 3-Phosphoglycerat-Dehydrogenase von *C. glutamicum* erzeugte Mutein SerAΔ197 funktionell ist, und mehr als 60 % der Wild Typ Aktivität aufweist.

In Tabelle 3 sind die Ergebnisse zusammengefasst.

**Tabelle 3: Überexpression des serA-Gens sowie der C-terminal verkürzten serA-Allele.**

| Stämme | spez. PGD-Aktivität [U/mg Protein] | Faktor der Überexpression |
|---|---|---|
| 13032pZ1 | 130 | 1.0 |
| 13032pZ1*serA* | 2140 | 16.5 |
| 13032pZ1*serA*Δ79 | 190 | 1.5 |
| 13032pZ1*serA*Δ188 | 440 | 3.4 |
| 13032pZ1*serA*Δ197 | 1320 | 10.0 |
| 13032pZ1*serA*Δ205 | 390 | 3.0 |
| 13032pZ1*serA*Δ211 | 150 | 1.2 |

| | | |
|---|---|---|
| * Die 3-Phosphoglycerat-Dehydrogenase-Aktivität im Stamm 13032pZ1 wurde auf 1,0 normiert | | |

### 3. Untersuchungen zur Inhibition der Wild Typ 3-Phosphoglycerat-Dehydrogenase aus C. glutamicum und des C-terminal verkürzten Muteins SerAΔ197 durch L-Serin

Im Folgenden wurde getestet, ob das um den C-Terminus verkürzte Mutein *SerA*Δ197 nicht mehr durch L-Serin gehemmt werden kann. Dazu wurde zunächst die Hemmbarkeit der 3-Phosphoglycerat-Dehydrogenase des Wild Typs in zellfreien Extrakten von *C. glutamicum* durch L-Serin anhand des oben beschriebenen Enzymtests untersucht. Hierzu wurden dem Testansatz zusätzlich 1, 5 und 10 mM L-Serin zugesetzt und 5 Minuten bei 30°C inkubiert. Die Reaktion wurde dann durch Zugabe von 15 mM 3-Phosphoglycerat gestartet. Die Inkubation war notwendig um eine Hemmung nachweisen zu können (Tab. 4). Diese Zeitabhängigkeit der L-Serin-Hemmung, die mehrere Minuten Inkubation benötigt, bevor ein konstanter Level der Inhibition erreicht wird, wurde auch schon für andere 3-Phosphoglycerat-Dehydrogenasen, z. B. für das aufgereinigte Enzym von *B. subtilis* beschrieben (Saski R. und Pitzer L., 1975, Eur. J. Biochem., 51:415-427).

**Tabelle 4: Inhibition der Wild Typ 3-Phosphoglycerat-Dehydrogenase von C. glutamicum durch L-Serin**

| L-Serin [mM] | relative 3-Phosphoglycerat-Dehydrogenase Aktivität [%] | |
|---|---|---|
| | ohne Inkubation | 5-minütige Inkubation bei 30°C |
| 0 | 100* | 100* |
| 1 | 106 | 96 |
| 5 | 112 | 82 |
| 10 | 104 | 56 |

| | | |
|---|---|---|
| * Die Aktivität der 3-Phosphoglycerat-Dehydrogenase ohne Zusatz von L-Serin wurde auf 100% gesetzt. | | |

Auf diesem Ergebnis aufbauend wurde die L-Serin-Inhibition der 3-Phopshoglycerat-Dehydrogenase in den Stämmen 13032pZ1*serA* und 13032pZ1*serA*Δ197 untersucht. Es zeigte sich, dass tatsächlich das C-terminal verkürzte 3-Phosphoglycerat-Dehydrogenase-Mutein nicht mehr signifikant durch L-Serin gehemmt werden kann (Tab. 5).

**Tabelle 5: Inhibition der überexprimierten 3-Phopshoglycerat-Dehydrogenase durch L-Serin in den Stämmen 13032pZ1serA und 13032pZ1serAΔ197**

| L-Serin [mM] | relative 3-Phosphoglycerat-Dehydrogenase Aktivität [%] ** | |
|---|---|---|
| | 13032pZ1*serA* | 13032pZ1*serA*Δ197 |
| 0 | 100* | 100* |
| 10 | 34 | 95 |

| | | |
|---|---|---|
| * Die Aktivität der 3-Phosphoglycerat-Dehydrogenase ohne Zusatz von L-Serin wurde auf 100% gesetzt ** Bestimmung der Aktivität nach 5-minütiger Inkubation bei 30°C mit und ohne L-Serin | | |

Damit ist es gelungen, durch Deletion des C-Terminus der 3-Phosphoglycerat-Dehydrogenase von *C. glutamicum* gezielt ein dereguliertes 3-Phosphoglycerat-Dehydrogenase-Mutein zu generieren.

### 4. Gesteigerte Akkumulation von L-Serin durch Überexpression des Gens für die deregulierte 3-Phosphoglycerat-Dehydrogenase (serAΔ197)

Zur Analyse der L-Serinausscheidung des Stammes mit deregulierter 3-Phosphoglycerat-Dehydrogenase wurden die Plasmide pZ1, pZ1*serA* und pZ1*serA*Δ197 in den Stamm Corynebacterium glutamicum 13032ΔpanBC transformiert (E. Radmacher, A. Vaitsikova, U. Burger, K. Krumbach, H. Sahm, L. Eggeling, 2002, Appl. Environ. Microbiol. (Publikation in Vorbereitung)). Dieser Stamm ist durch die Deletion der Pantothenat-Biosynthese Gene *panB* und *panC* Pantothenat-auxotroph, und zeichnet sich dadurch aus, dass er unter Pantothenat-Limitation aufgrund einer verstärkten Akkumulation von Pyruvat ca. 50 mM Alanin und 8 mM Valin ausscheidet. Darüberhinaus bildet der Stamm ca. 100 µM L-Serin und eignet sich somit als Ausgangsstamm für die Konstruktion eines L-Serin-Produzenten. Der Stamm mit dem Plasmid pZ1*serA* transformierte Stamm 13032ApanBCpZ1*serA* wurde gemäß Budapester Vertrag am 11.04.2002 bei der DSMZ unter der DSM Nr. 14922 hinterlegt.

Zur Untersuchung der L-Serinausscheidung wurden die drei Stämme in Komplexmedium (CgIII mit 2% Glukose und mit 50 µg/l Kanamycin) gezüchtet, und das Fermentationsmedium CGXII (J Bacteriol (1993) 175: 5595-5603) jeweils aus den Vorkulturen beimpft. Das Medium enthielt zusätzlich 50 µg/l Kanamycin und 1 µM Pantothenat. Es wurden zwei unabhängige Fermentationen durchgeführt. Nach Kultivierung für 24 bzw. 35 Stunden bei 30°C auf dem Rotationsschüttler bei 120 Upm wurde die in das Medium akkumulierte L-Serinmenge bestimmt. Die Bestimmung der Aminosäurekonzentration erfolgte mittels Hochdruckflüssigkeitchromatographie (J Chromat (1983) 266: 471-482). Das Ergebnis der Fermentation ist in Tabelle 6 dargestellt, und es zeigt sich, daß schon die Überexpression des Wildtyp serA-Gens eine ca. 10%ige Steigerung der L-Serin-Akkumulation im Medium hervorruft. Die Überexpression der deregulierten 3-Phosphoglycerat-Dehydrogenase erzielt dagegen sogar eine Steigerung von bis zu 40% im Vergleich zum Kontrollstamm der nur das Leerplasmid trägt. Somit stellt die Nutzung des konstruierten und beschriebenen Gens für das deregulierte L-Serin-Biosynthese Enzym 3-Phosphoglycerat-Dehydrogenase ein Verfahren dar, um die L-Serinbildung entscheidend zu verbessern.

**Tabelle 6: Akkumulation von L-Serin im Kulturüberstand von Corynebacterium glutamicum 13032ΔpanBC nach Expression der Gene serA bzw. serAΔ197**

| Stamm | t [h] | TG [mg/ml] | L-Serin [µM] | L-Serin/TG [mg/g] |
|---|---|---|---|---|
| 13032DpanBCpZ1 | 24 | 18,3 | 164 | 0,9 |
| 13032DpanBCpZ1*serA* | 24 | 14,7 | 163 | 1,2 |
| 13032DpanBCpZ1*serA*Δ197 | 24 | 16,5 | 199 | 1,3 |

| | | | | |
|---|---|---|---|---|
| * TG = Zelltrockengewicht | | | | |

## Patentansprüche

1. Nukleinsäure codierend für eine deregulierte 3-Phosphoglycerat-Dehydrogenase enthaltend ein Gen *ser*A gemäß SEQ ID No 1.

2. Nukleinsäure nach Anspruch 1,
**dadurch gekennzeichnet,**
**dass** sie aus coryneformen Bakterien isoliert werden.

3. Nukleinsäure nach einem der Ansprüche 1 oder 2,
**dadurch gekennzeichnet,**
**dass** sie aus Corynebacterium oder Brevibacterium isoliert werden.

4. Nukleinsäure nach einem der Ansprüche 1 bis 3,
**dadurch gekennzeichnet,**
**dass** sie aus Corynebacterium glutamicum oder Brevibacterium flavum isoliert werden.

5. Genstruktur enthaltend wenigstens eine Nukleotidsequenz gemäß den Ansprüchen 1 bis 4 sowie mit diesen operativ verknüpfte regulatorische Sequenzen.

6. Vektor enthaltend wenigstens eine Nukleotidsequenz gemäß Anspruch 1 bis 4 oder eine Genstruktur gemäß Anspruch 5 sowie zusätzliche Nukleotidsequenzen zur Selektion, zur Replikation in der Wirtszelle oder zur Integration in das Wirtszell-Genom.

7. Deregulierte 3-Phosphoglycerat-Dehydrogenase mit einer Aminosäuresequenz,
gemäß SEQ ID No 7.

8. Polypeptid nach Anspruch 7,
**dadurch gekennzeichnet,**
**dass** es aus coryneformen Bakterien stammt.

9. Polypeptid nach einem der Ansprüche 7 oder 8,
**dadurch gekennzeichnet,**
**dass** es aus Corynebacterium oder Brevibacterium stammt.

10. Polypeptid nach einem der Ansprüche 7 bis 9,
**dadurch gekennzeichnet,**
**dass** es aus Corynebacterium glutamicum oder Brevibacterium flavum stammt.

11. Mikroorganismus enthaltend wenigstens eine Nukleinsäure gemäß Anspruch 1 bis 4 in replizierbarer Form, welche im Vergleich zum Wild Typ Mikroorganismus verstärkt exprimiert wird und/oder deren Kopienzahl erhöht ist.

12. Mikroorganismus gemäß Anspruch 11 enthaltend in replizierbarer Form eine Genstruktur gemäß Anspruch 5 oder einen Vektor gemäß Anspruch 6.

13. Mikroorganismus gemäß einem der Ansprüche 11 oder 12 enthaltend wenigstens ein Polypeptid gemäß Anspruch 8 bis 10, welcher eine im Vergleich zu dem entsprechenden Wild Typ Stamm aktive deregulierte 3-Phosphoglycerat-Dehydrogenase aufweist.

14. Mikroorganismus gemäß einem der Ansprüche 11 bis 13,
**dadurch gekennzeichnet,**
**dass** er ein coryneformes Bakterium ist.

15. Mikroorganismus gemäß einem der Ansprüche 11 bis 14,
**dadurch gekennzeichnet,**
**dass** er zur Gattung Corynebacterium oder Brevibacterium gehört.

16. Mikroorganismus gemäß einem der Ansprüche 11 bis 15,
**dadurch gekennzeichnet,**
**dass** er zu Corynebacterium glutamicum oder Brevibacterium flavum gehört.

17. Verfahren zur mikrobiellen Herstellung von L-Serin,
**dadurch gekennzeichnet, dass**
a) wenigstens eine Nukleinsäure gemäß einem der Ansprüche 1 bis 4 isoliert aus einem coryneformen Bakterium in einen Mikroorganismus übertragen wird und dort exprimiert wird, wobei die Genexpression und/oder die Aktivität des entsprechend codierten Polypeptids gegenüber dem entsprechend nicht genetisch veränderten Mikroorganismus erhöht ist,
b) dieser genetisch veränderte Mikroorganismus aus Schritt b) zur mikrobiellen Herstellung eingesetzt wird und
c) das entsprechend gebildete L- Serin aus dem Kulturmedium isoliert wird.

## Claims

1. Nucleic acids that encode a deregulated 3-phosphoglycerate dehydrogenase containing a serA gene according to SEQ ID No. 1.

2. Nucleic acids according to claim 1, **characterised in that** they are isolated from coryneform bacteria.

3. Nucleic acids according to either of claims 1 or 2, **characterised in that** they are isolated from *Corynebacterium* or *Brevibacterium.*

4. Nucleic acids according to one of claims 1 to 3, **characterised in that** they are isolated from *Corynebacterium glutamicum* or *Brevibacterium flavum.*

5. Gene structure containing at least one nucleotide sequence according to claims 1 to 4, as well as regulatory sequences operatively linked thereto.

6. Vector containing at least one nucleotide sequence according to claims 1 to 4 or a gene structure according to claim 5, as well as additional nucleotide sequences for selection, replication in the host cell or integration into the host cell genome.

7. Deregulated 3-phosphoglycerate dehydrogenase with an amino acid sequence according to SEQ ID No. 7.

8. Polypeptide according to claim 7, **characterised in that** it is derived from coryneform bacteria.

9. Polypeptide according to either of claims 7 or 8, **characterised in that** it is derived from *Corynebacterium* or *Brevibacterium*.

10. Polypeptide according to one of claims 7 to 9, **characterised in that** it is derived from *Corynebacterium glutamicum* or *Brevibacterium flavum*.

11. Microorganism containing at least one nucleic acid according to claims 1 to 4 in replicable form expressed in an enhanced manner and/or with an increased copy number in comparison with the wild-type microorganism.

12. Microorganism according to claim 11 containing a gene structure according to claim 5 or a vector according to claim 6 in replicable form.

13. Microorganism according to either of claims 11 or 12 containing at least one polypeptide according to claims 8 to 10 comprising an active deregulated 3-phosphoglycerate dehydrogenase in comparison with the corresponding wild-type strain.

14. Microorganism according to one of claims 11 to 13, **characterised in that** it is a coryneform bacterium.

15. Microorganism according to one of claims 11 to 14, **characterised in that** it belongs to the genus *Corynebacterium* or *Brevibacterium.*

16. Microorgamsm according to one of claims 11 to 15, **characterised in that** it belongs to *Corynebacterium glutamicum* or *Brevibacterium flavum.*

17. Method for the microbial production of L-serine, **characterised in that**
a) at least one nucleic acid according to one of claims 1 to 4 isolated from a coryneform bacterium is transferred to a microorganism and expressed there, the gene expression and/or the activity of the correspondingly encoded polypeptide being increased in comparison with the correspondingly non-genetically modified microorganism,
b) this genetically modified microorganism from step a) is used for the microbial production and
c) the correspondingly formed L-serine is isolated from the culture medium.

## Revendications

1. Acide nucléique codant pour une 3-phosphoglycérate déshydrogénase dérégulée contenant un gène serA ayant la SEQ ID N° 1.

2. Acide nucléique selon la revendication 1, **caractérisé en ce**
**qu'**il est isolé à partir de bactéries corynéformes.

3. Acide nucléique selon l'une des revendications 1 ou 2,
**caractérisé en ce**
**qu'**il est isolé à partir des genres Corynebacterium ou Brevibacterium.

4. Acide nucléique selon l'une des revendications 1 à 3,
**caractérisé en ce**
**qu'**il est isolé à partir des espèces *Corynebacterium glutamicum* ou *Brevibacterium flavum.*

5. Structure génique contenant au moins une séquence nucléotidique selon les revendications 1 à 4 ainsi que des séquences régulatrices liées de manière opérante à ladite séquence nucléotidique.

6. Vecteur contenant au moins une séquence nucléotidique selon les revendications 1 à 4 ou une structure génique selon la revendication 5 ainsi que des séquences nucléotidiques supplémentaires pour la sélection, pour la réplication dans la cellule hôte ou pour l'intégration dans le génome de la cellule hôte.

7. 3-Phosphoglycérate déshydrogénase dérégulée ayant une séquence d'acides aminés de SEQ ID N° 7.

8. Polypeptide selon la revendication 7, **caractérisé en ce**
**qu'**il est issu de bactéries corynéformes.

9. Polypeptide selon l'une des revendications 7 ou 8,
**caractérisé en ce**
**qu'**il est issu des genres Corynebacterium ou Brevibacterium.

10. Polypeptide selon l'une des revendications 7 à 9,
**caractérisé en ce**
**qu'**il est issu des espèces *Corynebacterium glutamicum* ou *Brevibacterium flavum.*

11. Micro-organisme, contenant au moins un acide nucléique selon les revendications 1 à 4 sous une forme réplicable, dont l'expression est renforcée et/ou le nombre de copies augmenté par rapport au micro-organisme de type sauvage.

12. Micro-organisme selon la revendication 11, contenant sous une forme réplicable une structure génique selon la revendication 5 ou un vecteur selon la revendication 6.

13. Micro-organisme selon l'une des revendications 11 ou 12, contenant au moins un polypeptide selon les revendications 8 à 10, qui présente par rapport à la souche sauvage correspondante une 3-phosphoglycérate déshydrogénase dérégulée active.

14. Micro-organisme selon l'une des revendications 11 à 13,
**caractérisé en ce**
**qu'**il est une bactérie corynéforme.

15. Micro-organisme selon l'une des revendications 11 à 14,
**caractérisé en ce**
**qu'**il appartient au genre Corynebacterium ou Brevibacterium.

16. Micro-organisme selon l'une des revendications 11 à 15,
**caractérisé en ce**
**qu'**il appartient à l'espèce *Corynebacterium glutamicum* ou *Brevibacterium flavum.*

17. Procédé de production microbienne de L-sérine, **caractérisé en ce que**
a) au moins un acide nucléique selon l'une des revendications 1 à 4 isolé à partir d'une bactérie corynéforme est transféré dans un micro-organisme où il est exprimé, l'expression génique et/ou l'activité du polypeptide codé correspondant étant augmentée par rapport au micro-organisme correspondant non modifié génétiquement,
b) le micro-organisme génétiquement modifié de l'étape a) est utilisé pour la production microbienne, et
c) la L-sérine ainsi produite est isolée à partir du milieu de culture.


### SEQUENZPROTOKOLL

<110> Forschungszentrum Jülich GmbH
<120> Nukleotidsequenzen coryneformer Bakterien kodierend für an der Biosynthese von L-Serin beteiligte Proteine sowie Verfahren zur Herstellung von L-Serin
<130> 1.2002
<140>
   <141>
<160> 12
<170> PatentIn Ver. 2.1
<210> 1
   <211> 1253
   <212> DNA
   <213> Corynebacterium glutamicum
<400> 1
   tctagagccg gagacgtgaa taaaattcgc agctcattcc atcagcgtaa acgcagcttt 60
   ttgcatggtg agacaccttt gggggtaaat ctcacagcat gaatctctgg gttagatgac 120
   tttctgggtg ggggagggtt tagaatgttt ctagtcgcac gccaaaaccc ggcgtggaca 180
   cgtctgcagc cgacgcggtc gtgcctgttg tagacggaca ttcctagttt ttccaggagt 240
   aacttgtgag ccagaatggc cgtccggtag tcctcatcgc cgataagctt gcgcagtcca 300
   ctgttgacgc gcttggagat gcagtagaag tccgttgggt tgacggacct aaccgcccag 360
   aactgcttga tgcagttaag gaagcggacg cactgctcgt gcgttctgct accactgtcg 420
   atgctgaagt catcgccgct gcccctaact tgaagatcgt cggtcgtgcc ggcgtgggct 480
   tggacaacgt tgacatccct gctgccactg aagctggcgt catggttgct aacgcaccga 540
   cctctaatat tcactccgct tgtgagcacg caatttcttt gctgctgtct actgctcgcc 600
   agatccctgc tgctgatgcg acgctgcgtg agggcgagtg gaagcggtct tctttcaacg 660
   gtgtggaaat tttcggaaaa actgtcggta tcgtcggttt tggccacatt ggtcagttgt 720
   ttgctcagcg tcttgctgcg tttgagacca ccattgttgc ttacgatcct tacgctaacc 780
   ctgctcgtgc ggctcagctg aacgttgagt tggttgagtt ggatgagctg atgagccgtt 840
   ctgactttgt caccattcac cttcctaaga ccaaggaaac tgctggcatg tttgatgcgc 900
   agctccttgc taagtccaag aagggccaga tcatcatcaa cgctgctcgt ggtggccttg 960
   ttgatgagca ggctttggct gatgcgattg agtccggtca cattcgtggc gctggtttcg 1020
   atgtgtactc caccgagcct tgcactgatt ctcctttgtt caagttgcct caggttgttg 1080
   tgactcctca cttgggtgct tctactgaag aggctcagga tcgtgcgggt actgacgttg 1140
   ctgattctgt gctcaaggcg ctggctggcg agttcgtggc ggatgctgtg aacgtttccg 1200
   gtggtcgcgt gggcgaagag gttgctgtgt ggatggatct ggcttaagga tcc        1253
<210> 2
   <211> 1607
   <212> DNA
   <213> Corynebacterium glutamicum
<400> 2
   tctagagccg gagacgtgaa taaaattcgc agctcattcc atcagcgtaa acgcagcttt 60
   ttgcatggtg agacaccttt gggggtaaat ctcacagcat gaatctctgg gttagatgac 120
   tttctgggtg ggggagggtt tagaatgttt ctagtcgcac gccaaaaccc ggcgtggaca 180
   cgtctgcagc cgacgcggtc gtgcctgttg tagacggaca ttcctagttt ttccaggagt 240
   aacttgtgag ccagaatggc cgtccggtag tcctcatcgc cgataagctt gcgcagtcca 300
   ctgttgacgc gcttggagat gcagtagaag tccgttgggt tgacggacct aaccgcccag 360
   aactgcttga tgcagttaag gaagcggacg cactgctcgt gcgttctgct accactgtcg 420
   atgctgaagt catcgccgct gcccctaact tgaagatcgt cggtcgtgcc ggcgtgggct 480
   tggacaacgt tgacatccct gctgccactg aagctggcgt catggttgct aacgcaccga 540
   cctctaatat tcactccgct tgtgagcacg caatttcttt gctgctgtct actgctcgcc 600
   agatccctgc tgctgatgcg acgctgcgtg agggcgagtg gaagcggtct tctttcaacg 660
   gtgtggaaat tttcggaaaa actgtcggta tcgtcggttt tggccacatt ggtcagttgt 720
   ttgctcagcg tcttgctgcg tttgagacca ccattgttgc ttacgatcct tacgctaacc 780
   ctgctcgtgc ggctcagctg aacgttgagt tggttgagtt ggatgagctg atgagccgtt 840
   ctgactttgt caccattcac cttcctaaga ccaaggaaac tgctggcatg tttgatgcgc 900
   agctccttgc taagtccaag aagggccaga tcatcatcaa cgctgctcgt ggtggccttg 960
   ttgatgagca ggctttggct gatgcgattg agtccggtca cattcgtggc gctggtttcg 1020
   atgtgtactc caccgagcct tgcactgatt ctcctttgtt caagttgcct caggttgttg 1080
   tgactcctca cttgggtgct tctactgaag aggctcagga tcgtgcgggt actgacgttg 1140
   ctgattctgt gctcaaggcg ctggctggcg agttcgtggc ggatgctgtg aacgtttccg 1200
   gtggtcgcgt gggcgaagag gttgctgtgt ggatggatct ggctcgcaag cttggtcttc 1260
   ttgctggcaa gcttgtcgac gccgccccag tctccattga ggttgaggct cgaggcgagc 1320
   tttcttccga gcaggtcgat gcacttggtt tgtccgctgt tcgtggtttg ttctccggaa 1380
   ttatcgaaga gtccgttact ttcgtcaacg ctcctcgcat tgctgaagag cgtggcctgg 1440
   acatctccgt gaagaccaac tctgagtctg ttactcaccg ttccgtcctg caggtcaagg 1500
   tcattactgg cagcggcgcg agcgcaactg ttgttggtgc cctgactggt cttgagcgcg 1560
   ttgagaagat cacccgcatc aatggccgtg gcctggatta aggatcc             1607
<210> 3
   <211> 1280
   <212> DNA
   <213> Corynebacterium glutamicum
<400> 3
   tctagagccg gagacgtgaa taaaattcgc agctcattcc atcagcgtaa acgcagcttt 60
   ttgcatggtg agacaccttt gggggtaaat ctcacagcat gaatctctgg gttagatgac 120
   tttctgggtg ggggagggtt tagaatgttt ctagtcgcac gccaaaaccc ggcgtggaca 180
   cgtctgcagc cgacgcggtc gtgcctgttg tagacggaca ttcctagttt ttccaggagt 240
   aacttgtgag ccagaatggc cgtccggtag tcctcatcgc cgataagctt gcgcagtcca 300
   ctgttgacgc gcttggagat gcagtagaag tccgttgggt tgacggacct aaccgcccag 360
   aactgcttga tgcagttaag gaagcggacg cactgctcgt gcgttctgct accactgtcg 420
   atgctgaagt catcgccgct gcccctaact tgaagatcgt cggtcgtgcc ggcgtgggct 480
   tggacaacgt tgacatccct gctgccactg aagctggcgt catggttgct aacgcaccga 540
   cctctaatat tcactccgct tgtgagcacg caatttcttt gctgctgtct actgctcgcc 600
   agatccctgc tgctgatgcg acgctgcgtg agggcgagtg gaagcggtct tctttcaacg 660
   gtgtggaaat tttcggaaaa actgtcggta tcgtcggttt tggccacatt ggtcagttgt 720
   ttgctcagcg tcttgctgcg tttgagacca ccattgttgc ttacgatcct tacgctaacc 780
   ctgctcgtgc ggctcagctg aacgttgagt tggttgagtt ggatgagctg atgagccgtt 840
   ctgactttgt caccattcac cttcctaaga ccaaggaaac tgctggcatg tttgatgcgc 900
   agctccttgc taagtccaag aagggccaga tcatcatcaa cgctgctcgt ggtggccttg 960
   ttgatgagca ggctttggct gatgcgattg agtccggtca cattcgtggc gctggtttcg 1020
   atgtgtactc caccgagcct tgcactgatt ctcctttgtt caagttgcct caggttgttg 1080
   tgactcctca cttgggtgct tctactgaag aggctcagga tcgtgcgggt actgacgttg 1140
   ctgattctgt gctcaaggcg ctggctggcg agttcgtggc ggatgctgtg aacgtttccg 1200
   gtggtcgcgt gggcgaagag gttgctgtgt ggatggatct ggctcgcaag cttggtcttc 1260
   ttgctggcaa gtaaggatcc                                             1280
<210> 4
   <211> 1229
   <212> DNA
   <213> Corynebacterium glutamicum
<400> 4
   tctagagccg gagacgtgaa taaaattcgc agctcattcc atcagcgtaa acgcagcttt 60
   ttgcatggtg agacaccttt gggggtaaat ctcacagcat gaatctctgg gttagatgac 120
   tttctgggtg ggggagggtt tagaatgttt ctagtcgcac gccaaaaccc ggcgtggaca 180
   cgtctgcagc cgacgcggtc gtgcctgttg tagacggaca ttcctagttt ttccaggagt 240
   aacttgtgag ccagaatggc cgtccggtag tcctcatcgc cgataagctt gcgcagtcca 300
   ctgttgacgc gcttggagat gcagtagaag tccgttgggt tgacggacct aaccgcccag 360
   aactgcttga tgcagttaag gaagcggacg cactgctcgt gcgttctgct accactgtcg 420
   atgctgaagt catcgccgct gcccctaact tgaagatcgt cggtcgtgcc ggcgtgggct 480
   tggacaacgt tgacatccct gctgccactg aagctggcgt catggttgct aacgcaccga 540
   cctctaatat tcactccgct tgtgagcacg caatttcttt gctgctgtct actgctcgcc 600
   agatccctgc tgctgatgcg acgctgcgtg agggcgagtg gaagcggtct tctttcaacg 660
   gtgtggaaat tttcggaaaa actgtcggta tcgtcggttt tggccacatt ggtcagttgt 720
   ttgctcagcg tcttgctgcg tttgagacca ccattgttgc ttacgatcct tacgctaacc 780
   ctgctcgtgc ggctcagctg aacgttgagt tggttgagtt ggatgagctg atgagccgtt 840
   ctgactttgt caccattcac cttcctaaga ccaaggaaac tgctggcatg tttgatgcgc 900
   agctccttgc taagtccaag aagggccaga tcatcatcaa cgctgctcgt ggtggccttg 960
   ttgatgagca ggctttggct gatgcgattg agtccggtca cattcgtggc gctggtttcg 1020
   atgtgtactc caccgagcct tgcactgatt ctcctttgtt caagttgcct caggttgttg 1080
   tgactcctca cttgggtgct tctactgaag aggctcagga tcgtgcgggt actgacgttg 1140
   ctgattctgt gctcaaggcg ctggctggcg agttcgtggc ggatgctgtg aacgtttccg 1200
   gtggtcgcgt gggcgaagag taaggatcc                                  1229
<210> 5
   <211> 1211
   <212> DNA
   <213> Corynebacterium glutamicum
<400> 5
   tctagagccg gagacgtgaa taaaattcgc agctcattcc atcagcgtaa acgcagcttt 60
   ttgcatggtg agacaccttt gggggtaaat ctcacagcat gaatctctgg gttagatgac 120
   tttctgggtg ggggagggtt tagaatgttt ctagtcgcac gccaaaaccc ggcgtggaca 180
   cgtctgcagc cgacgcggtc gtgcctgttg tagacggaca ttcctagttt ttccaggagt 240
   aacttgtgag ccagaatggc cgtccggtag tcctcatcgc cgataagctt gcgcagtcca 300
   ctgttgacgc gcttggagat gcagtagaag tccgttgggt tgacggacct aaccgcccag 360
   aactgcttga tgcagttaag gaagcggacg cactgctcgt gcgttctgct accactgtcg 420
   atgctgaagt catcgccgct gcccctaact tgaagatcgt cggtcgtgcc ggcgtgggct 480
   tggacaacgt tgacatccct gctgccactg aagctggcgt catggttgct aacgcaccga 540
   cctctaatat tcactccgct tgtgagcacg caatttcttt gctgctgtct actgctcgcc 600
   agatccctgc tgctgatgcg acgctgcgtg agggcgagtg gaagcggtct tctttcaacg 660
   gtgtggaaat tttcggaaaa actgtcggta tcgtcggttt tggccacatt ggtcagttgt 720
   ttgctcagcg tcttgctgcg tttgagacca ccattgttgc ttacgatcct tacgctaacc 780
   ctgctcgtgc ggctcagctg aacgttgagt tggttgagtt ggatgagctg atgagccgtt 840
   ctgactttgt caccattcac cttcctaaga ccaaggaaac tgctggcatg tttgatgcgc 900
   agctccttgc taagtccaag aagggccaga tcatcatcaa cgctgctcgt ggtggccttg 960
   ttgatgagca ggctttggct gatgcgattg agtccggtca cattcgtggc gctggtttcg 1020
   atgtgtactc caccgagcct tgcactgatt ctcctttgtt caagttgcct caggttgttg 1080
   tgactcctca cttgggtgct tctactgaag aggctcagga tcgtgcgggt actgacgttg 1140
   ctgattctgt gctcaaggcg ctggctggcg agttcgtggc ggatgctgtg aacgtttccg 1200
   gttaaggatc c                                                      1211
<210> 6
   <211> 2043
   <212> DNA
   <213> Corynebacterium glutamicum
<400> 6
   tctagagccg gagacgtgaa taaaattcgc agctcattcc atcagcgtaa acgcagcttt 60
   ttgcatggtg agacaccttt gggggtaaat ctcacagcat gaatctctgg gttagatgac 120
   tttctgggtg ggggagggtt tagaatgttt ctagtcgcac gccaaaaccc ggcgtggaca 180
   cgtctgcagc cgacgcggtc gtgcctgttg tagacggaca ttcctagttt ttccaggagt 240
   aacttgtgag ccagaatggc cgtccggtag tcctcatcgc cgataagctt gcgcagtcca 300
   ctgttgacgc gcttggagat gcagtagaag tccgttgggt tgacggacct aaccgcccag 360
   aactgcttga tgcagttaag gaagcggacg cactgctcgt gcgttctgct accactgtcg 420
   atgctgaagt catcgccgct gcccctaact tgaagatcgt cggtcgtgcc ggcgtgggct 480
   tggacaacgt tgacatccct gctgccactg aagctggcgt catggttgct aacgcaccga 540
   cctctaatat tcactccgct tgtgagcacg caatttcttt gctgctgtct actgctcgcc 600
   agatccctgc tgctgatgcg acgctgcgtg agggcgagtg gaagcggtct tctttcaacg 660
   gtgtggaaat tttcggaaaa actgtcggta tcgtcggttt tggccacatt ggtcagttgt 720
   ttgctcagcg tcttgctgcg tttgagacca ccattgttgc ttacgatcct tacgctaacc 780
   ctgctcgtgc ggctcagctg aacgttgagt tggttgagtt ggatgagctg atgagccgtt 840
   ctgactttgt caccattcac cttcctaaga ccaaggaaac tgctggcatg tttgatgcgc 900
   agctccttgc taagtccaag aagggccaga tcatcatcaa cgctgctcgt ggtggccttg 960
   ttgatgagca ggctttggct gatgcgattg agtccggtca cattcgtggc gctggtttcg 1020
   atgtgtactc caccgagcct tgcactgatt ctcctttgtt caagttgcct caggttgttg 1080
   tgactcctca cttgggtgct tctactgaag aggctcagga tcgtgcgggt actgacgttg 1140
   ctgattctgt gctcaaggcg ctggctggcg agttcgtggc ggatgctgtg aacgtttccg 1200
   gtggtcgcgt gggcgaagag gttgctgtgt ggatggatct ggctcgcaag cttggtcttc 1260
   ttgctggcaa gcttgtcgac gccgccccag tctccattga ggttgaggct cgaggcgagc 1320
   tttcttccga gcaggtcgat gcacttggtt tgtccgctgt tcgtggtttg ttctccggaa 1380
   ttatcgaaga gtccgttact ttcgtcaacg ctcctcgcat tgctgaagag cgtggcctgg 1440
   acatctccgt gaagaccaac tctgagtctg ttactcaccg ttccgtcctg caggtcaagg 1500
   tcattactgg cagcggcgcg agcgcaactg ttgttggtgc cctgactggt cttgagcgcg 1560
   ttgagaagat cacccgcatc aatggccgtg gcctggatct gcgcgcagag ggtctgaacc 1620
   tcttcctgca gtacactgac gctcctggtg cactgggtac cgttggtacc aagctgggtg 1680
   ctgctggcat caacatcgag gctgctgcgt tgactcaggc tgagaagggt gacggcgctg 1740
   tcctgatcct gcgtgttgag tccgctgtct ctgaagagct ggaagctgaa atcaacgctg 1800
   agttgggtgc tacttccttc caggttgatc ttgactaatt agagatccat ttgcttgaac 1860
   cgccttccca tctttgaatt cattcaaggt ggtaaggcgg ttttcgctct tttaatacag 1920
   ttttaaaggt agatttggga gagaagattt cccttaagaa aggttcttaa caaccatgcc 1980
   gcctgcgacg ctgttcaatg ttttgacttc agctggactt gaccctcacc agtctaagga 2040
   tcc                                                               2043
<210> 7
   <211> 333
   <212> PRT
   <212> Corynebacterium glutamicum
<400> 7
   Met Ser Gln Asn Gly Arg Pro Val Val Leu Ile Ala Asp Lys Leu Ala
   1               5                   10                  15
   Gln Ser Thr Val Asp Ala Leu Gly Asp Ala Val Glu Val Arg Trp Val
               20                  25                  30
   Asp Gly Pro Asn Arg Pro Glu Leu Leu Asp Ala Val Lys Glu Ala Asp
           35                  40                  45
   Ala Leu Leu Val Arg Ser Ala Thr Thr Val Asp Ala Glu Val Ile Ala
       50                  55                  60
   Ala Ala Pro Asn Leu Lys Ile Val Gly Arg Ala Gly Val Gly Leu Asp
   65                  70                  75                  80
   Asn Val Asp Ile Pro Ala Ala Thr Glu Ala Gly Val Met Val Ala Asn
                   85                  90                  95
   Ala Pro Thr Ser Asn Ile His Ser Ala Cys Glu His Ala Ile Ser Leu
               100                 105                 110
   Leu Leu Ser Thr Ala Arg Gln Ile Pro Ala Ala Asp Ala Thr Leu Arg
   115 120 125
   Glu Gly Glu Trp Lys Arg Ser Ser Phe Asn Gly Val Glu Ile Phe Gly
   130 135 140
   Lys Thr Val Gly Ile Val Gly Phe Gly His Ile Gly Gln Leu Phe Ala
   145 150 155 160
   Gln Arg Leu Ala Ala Phe Glu Thr Thr Ile Val Ala Tyr Asp Pro Tyr
   165 170 175
   Ala Asn Pro Ala Arg Ala Ala Gln Leu Asn Val Glu Leu Val Glu Leu
   180 185 190
   Asp Glu Leu Met Ser Arg Ser Asp Phe Val Thr Ile His Leu Pro Lys
   195 200 205
   Thr Lys Glu Thr Ala Gly Met Phe Asp Ala Gln Leu Leu Ala Lys Ser
   210 215 220
   Lys Lys Gly Gln Ile Ile Ile Asn Ala Ala Arg Gly Gly Leu Val Asp
   225 230 235 240
   Glu Gln Ala Leu Ala Asp Ala Ile Glu Ser Gly His Ile Arg Gly Ala
   245 250 255
   Gly Phe Asp Val Tyr Ser Thr Glu Pro Cys Thr Asp Ser Pro Leu Phe
   260 265 270
   Lys Leu Pro Gln Val Val Val Thr Pro His Leu Gly Ala Ser Thr Glu
   275 280 285
   Glu Ala Gln Asp Arg Ala Gly Thr Asp Val Ala Asp Ser Val Leu Lys
   290 295 300
   Ala Leu Ala Gly Glu Phe Val Ala Asp Ala Val Asn Val Ser Gly Gly
   305 310 315 320
   Arg Val Gly Glu Glu Val Ala Val Trp Met Asp Leu Ala
   325 330
<210> 8
   <211> 451
   <212> PRT
   <213> Corynebacterium glutamicum
<400> 8
   Met Ser Gln Asn Gly Arg Pro Val Val Leu Ile Ala Asp Lys Leu Ala
   1 5 10 15
   Gln Ser Thr Val Asp Ala Leu Gly Asp Ala Val Glu Val Arg Trp Val
   20 25 30
   Asp Gly Pro Asn Arg Pro Glu Leu Leu Asp Ala Val Lys Glu Ala Asp
   35 40 45
   Ala Leu Leu Val Arg Ser Ala Thr Thr Val Asp Ala Glu Val Ile Ala
   50 55 60
   Ala Ala Pro Asn Leu Lys Ile Val Gly Arg Ala Gly Val Gly Leu Asp
   65 70 75 80
   Asn Val Asp Ile Pro Ala Ala Thr Glu Ala Gly Val Met Val Ala Asn
   85 90 95
   Ala Pro Thr Ser Asn Ile His Ser Ala Cys Glu His Ala Ile Ser Leu
   100 105 110
   Leu Leu Ser Thr Ala Arg Gln Ile Pro Ala Ala Asp Ala Thr Leu Arg
   115 120 125
   Glu Gly Glu Trp Lys Arg Ser Ser Phe Asn Gly Val Glu Ile Phe Gly
   130 135 140
   Lys Thr Val Gly Ile Val Gly Phe Gly His Ile Gly Gln Leu Phe Ala
   145 150 155 160
   Gln Arg Leu Ala Ala Phe Glu Thr Thr Ile Val Ala Tyr Asp Pro Tyr
   165 170 175
   Ala Asn Pro Ala Arg Ala Ala Gln Leu Asn Val Glu Leu Val Glu Leu
   180 185 190
   Asp Glu Leu Met Ser Arg Ser Asp Phe Val Thr Ile His Leu Pro Lys
   195 200 205
   Thr Lys Glu Thr Ala Gly Met Phe Asp Ala Gln Leu Leu Ala Lys Ser
   210 215 220
   Lys Lys Gly Gln Ile Ile Ile Asn Ala Ala Arg Gly Gly Leu Val Asp
   225 230 235 240
   Glu Gln Ala Leu Ala Asp Ala Ile Glu Ser Gly His Ile Arg Gly Ala
   245 250 255
   Gly Phe Asp Val Tyr Ser Thr Glu Pro Cys Thr Asp Ser Pro Leu Phe
   260 265 270
   Lys Leu Pro Gln Val Val Val Thr Pro His Leu Gly Ala Ser Thr Glu
   275 280 285
   Glu Ala Gln Asp Arg Ala Gly Thr Asp Val Ala Asp Ser Val Leu Lys
   290 295 300
   Ala Leu Ala Gly Glu Phe Val Ala Asp Ala Val Asn Val Ser Gly Gly
   305 310 315 320
   Arg Val Gly Glu Glu Val Ala Val Trp Met Asp Leu Ala Arg Lys Leu
   325 330 335
   Gly Leu Leu Ala Gly Lys Leu Val Asp Ala Ala Pro Val Ser Ile Glu
   340 345 350
   Val Glu Ala Arg Gly Glu Leu Ser Ser Glu Gln Val Asp Ala Leu Gly
   355 360 365
   Leu Ser Ala Val Arg Gly Leu Phe Ser Gly Ile Ile Glu Glu Ser Val
   370 375 380
   Thr Phe Val Asn Ala Pro Arg Ile Ala Glu Glu Arg Gly Leu Asp Ile
   385 390 395 400
   Ser Val Lys Thr Asn Ser Glu Ser Val Thr His Arg Ser Val Leu Gln
   405 410 415
   Val Lys Val Ile Thr Gly Ser Gly Ala Ser Ala Thr Val Val Gly Ala
   420 425 430
   Leu Thr Gly Leu Glu Arg Val Glu Lys Ile Thr Arg Ile Asn Gly Arg
   435 440 445
   Gly Leu Asp
   450
<210> 9
   <211> 342
   <212> PRT
   <213> Corynebacterium glutamicum
<400> 9
   Met Ser Gln Asn Gly Arg Pro Val Val Leu Ile Ala Asp Lys Leu Ala
   1               5                   10                  15
   Gln Ser Thr Val Asp Ala Leu Gly Asp Ala Val Glu Val Arg Trp Val
               20                  25                  30
   Asp Gly Pro Asn Arg Pro Glu Leu Leu Asp Ala Val Lys Glu Ala Asp
           35                  40                  45
   Ala Leu Leu Val Arg Ser Ala Thr Thr Val Asp Ala Glu Val Ile Ala
       50                  55                  60
   Ala Ala Pro Asn Leu Lys Ile Val Gly Arg Ala Gly Val Gly Leu Asp
   65                  70                  75                  80
   Asn Val Asp Ile Pro Ala Ala Thr Glu Ala Gly Val Met Val Ala Asn
                   85                  90                  95
   Ala Pro Thr Ser Asn Ile His Ser Ala Cys Glu His Ala Ile Ser Leu
               100                 105                 110
   Leu Leu Ser Thr Ala Arg Gln Ile Pro Ala Ala Asp Ala Thr Leu Arg
           115                 120                 125
   Glu Gly Glu Trp Lys Arg Ser Ser Phe Asn Gly Val Glu Ile Phe Gly
       130                 135                 140
   Lys Thr Val Gly Ile Val Gly Phe Gly His Ile Gly Gln Leu Phe Ala
   145                 150                 155                 160
   Gln Arg Leu Ala Ala Phe Glu Thr Thr Ile Val Ala Tyr Asp Pro Tyr
                   165                 170                 175
   Ala Asn Pro Ala Arg Ala Ala Gln Leu Asn Val Glu Leu Val Glu Leu
               180                 185                 190
   Asp Glu Leu Met Ser Arg Ser Asp Phe Val Thr Ile His Leu Pro Lys
           195                 200                 205
   Thr Lys Glu Thr Ala Gly Met Phe Asp Ala Gln Leu Leu Ala Lys Ser
       210                 215                 220
   Lys Lys Gly Gln Ile Ile Ile Asn Ala Ala Arg Gly Gly Leu Val Asp
   225                 230                 235                 240
   Glu Gln Ala Leu Ala Asp Ala Ile Glu Ser Gly His Ile Arg Gly Ala
                   245                 250                 255
   Gly Phe Asp Val Tyr Ser Thr Glu Pro Cys Thr Asp Ser Pro Leu Phe
   260 265 270
   Lys Leu Pro Gln Val Val Val Thr Pro His Leu Gly Ala Ser Thr Glu
   275 280 285
   Glu Ala Gln Asp Arg Ala Gly Thr Asp Val Ala Asp Ser Val Leu Lys
   290 295 300
   Ala Leu Ala Gly Glu Phe Val Ala Asp Ala Val Asn Val Ser Gly Gly
   305 310 315 320
   Arg Val Gly Glu Glu Val Ala Val Trp Met Asp Leu Ala Arg Lys Leu
   325 330 335
   Gly Leu Leu Ala Gly Lys
   340
<210> 10
   <211> 325
   <212> PRT
   <213> Corynebacterium glutamicum
<400> 10
   Met Ser Gln Asn Gly Arg Pro Val Val Leu Ile Ala Asp Lys Leu Ala
   1 5 10 15
   Gln Ser Thr Val Asp Ala Leu Gly Asp Ala Val Glu Val Arg Trp Val
   20 25 30
   Asp Gly Pro Asn Arg Pro Glu Leu Leu Asp Ala Val Lys Glu Ala Asp
   35 40 45
   Ala Leu Leu Val Arg Ser Ala Thr Thr Val Asp Ala Glu Val Ile Ala
   50 55 60
   Ala Ala Pro Asn Leu Lys Ile Val Gly Arg Ala Gly Val Gly Leu Asp
   65 70 75 80
   Asn Val Asp Ile Pro Ala Ala Thr Glu Ala Gly Val Met Val Ala Asn
   85 90 95
   Ala Pro Thr Ser Asn Ile His Ser Ala Cys Glu His Ala Ile Ser Leu
   100 105 110
   Leu Leu Ser Thr Ala Arg Gln Ile Pro Ala Ala Asp Ala Thr Leu Arg
   115 120 125
   Glu Gly Glu Trp Lys Arg Ser Ser Phe Asn Gly Val Glu Ile Phe Gly
   130 135 140
   Lys Thr Val Gly Ile Val Gly Phe Gly His Ile Gly Gln Leu Phe Ala
   145 150 155 160
   Gln Arg Leu Ala Ala Phe Glu Thr Thr Ile Val Ala Tyr Asp Pro Tyr
   165 170 175
   Ala Asn Pro Ala Arg Ala Ala Gln Leu Asn Val Glu Leu Val Glu Leu
   180 185 190
   Asp Glu Leu Met Ser Arg Ser Asp Phe Val Thr Ile His Leu Pro Lys
   195 200 205
   Thr Lys Glu Thr Ala Gly Met Phe Asp Ala Gln Leu Leu Ala Lys Ser
   210 215 220
   Lys Lys Gly Gln Ile Ile Ile Asn Ala Ala Arg Gly Gly Leu Val Asp
   225 230 235 240
   Glu Gln Ala Leu Ala Asp Ala Ile Glu Ser Gly His Ile Arg Gly Ala
   245 250 255
   Gly Phe Asp Val Tyr Ser Thr Glu Pro Cys Thr Asp Ser Pro Leu Phe
   260 265 270
   Lys Leu Pro Gln Val Val Val Thr Pro His Leu Gly Ala Ser Thr Glu
   275 280 285
   Glu Ala Gln Asp Arg Ala Gly Thr Asp Val Ala Asp Ser Val Leu Lys
   290 295 300
   Ala Leu Ala Gly Glu Phe Val Ala Asp Ala Val Asn Val Ser Gly Gly
   305 310 315 320
   Arg Val Gly Glu Glu
   325
<210> 11
   <211> 319
   <212> PRT
   <213> Corynebacterium glutamicum
<400> 11
   Met Ser Gln Asn Gly Arg Pro Val Val Leu Ile Ala Asp Lys Leu Ala
   1               5                   10                  15
   Gln Ser Thr Val Asp Ala Leu Gly Asp Ala Val Glu Val Arg Trp Val
               20                  25                  30
   Asp Gly Pro Asn Arg Pro Glu Leu Leu Asp Ala Val Lys Glu Ala Asp
           35                  40                  45
   Ala Leu Leu Val Arg Ser Ala Thr Thr Val Asp Ala Glu Val Ile Ala
       50                  55                  60
   Ala Ala Pro Asn Leu Lys Ile Val Gly Arg Ala Gly Val Gly Leu Asp
   65                  70                  75                  80
   Asn Val Asp Ile Pro Ala Ala Thr Glu Ala Gly Val Met Val Ala Asn
                   85                  90                  95
   Ala Pro Thr Ser Asn Ile His Ser Ala Cys Glu His Ala Ile Ser Leu
               100                 105                 110
   Leu Leu Ser Thr Ala Arg Gln Ile Pro Ala Ala Asp Ala Thr Leu Arg
           115                 120                 125
   Glu Gly Glu Trp Lys Arg Ser Ser Phe Asn Gly Val Glu Ile Phe Gly
       130                 135                 140
   Lys Thr Val Gly Ile Val Gly Phe Gly His Ile Gly Gln Leu Phe Ala
   145                 150                 155                 160
   Gln Arg Leu Ala Ala Phe Glu Thr Thr Ile Val Ala Tyr Asp Pro Tyr
                   165                 170                 175
   Ala Asn Pro Ala Arg Ala Ala Gln Leu Asn Val Glu Leu Val Glu Leu
               180                 185                 190
   Asp Glu Leu Met Ser Arg Ser Asp Phe Val Thr Ile His Leu Pro Lys
           195                 200                 205
   Thr Lys Glu Thr Ala Gly Met Phe Asp Ala Gln Leu Leu Ala Lys Ser
       210                 215                 220
   Lys Lys Gly Gln Ile Ile Ile Asn Ala Ala Arg Gly Gly Leu Val Asp
   225                 230                 235                 240
   Glu Gln Ala Leu Ala Asp Ala Ile Glu Ser Gly His Ile Arg Gly Ala
                   245                 250                 255
   Gly Phe Asp Val Tyr Ser Thr Glu Pro Cys Thr Asp Ser Pro Leu Phe
   260 265 270
   Lys Leu Pro Gln Val Val Val Thr Pro His Leu Gly Ala Ser Thr Glu
   275 280 285
   Glu Ala Gln Asp Arg Ala Gly Thr Asp Val Ala Asp Ser Val Leu Lys
   290 295 300
   Ala Leu Ala Gly Glu Phe Val Ala Asp Ala Val Asn Val Ser Gly
   305 310 315
<210> 12
   <211> 530
   <212> PRT
   <213> Corynebacterium glutamicum
<400> 12
   Met Ser Gln Asn Gly Arg Pro Val Val Leu Ile Ala Asp Lys Leu Ala
   1 5 10 15
   Gln Ser Thr Val Asp Ala Leu Gly Asp Ala Val Glu Val Arg Trp Val
   20 25 30
   Asp Gly Pro Asn Arg Pro Glu Leu Leu Asp Ala Val Lys Glu Ala Asp
   35 40 45
   Ala Leu Leu Val Arg Ser Ala Thr Thr Val Asp Ala Glu Val Ile Ala
   50 55 60
   Ala Ala Pro Asn Leu Lys Ile Val Gly Arg Ala Gly Val Gly Leu Asp
   65 70 75 80
   Asn Val Asp Ile Pro Ala Ala Thr Glu Ala Gly Val Met Val Ala Asn
   85 90 95
   Ala Pro Thr Ser Asn Ile His Ser Ala Cys Glu His Ala Ile Ser Leu
   100 105 110
   Leu Leu Ser Thr Ala Arg Gln Ile Pro Ala Ala Asp Ala Thr Leu Arg
   115 120 125
   Glu Gly Glu Trp Lys Arg Ser Ser Phe Asn Gly Val Glu Ile Phe Gly
   130 135 140
   Lys Thr Val Gly Ile Val Gly Phe Gly His Ile Gly Gln Leu Phe Ala
   145 150 155 160
   Gln Arg Leu Ala Ala Phe Glu Thr Thr Ile Val Ala Tyr Asp Pro Tyr
   165 170 175
   Ala Asn Pro Ala Arg Ala Ala Gln Leu Asn Val Glu Leu Val Glu Leu
   180 185 190
   Asp Glu Leu Met Ser Arg Ser Asp Phe Val Thr Ile His Leu Pro Lys
   195 200 205
   Thr Lys Glu Thr Ala Gly Met Phe Asp Ala Gln Leu Leu Ala Lys Ser
   210 215 220
   Lys Lys Gly Gln Ile Ile Ile Asn Ala Ala Arg Gly Gly Leu Val Asp
   225 230 235 240
   Glu Gln Ala Leu Ala Asp Ala Ile Glu Ser Gly His Ile Arg Gly Ala
   245 250 255
   Gly Phe Asp Val Tyr Ser Thr Glu Pro Cys Thr Asp Ser Pro Leu Phe
   260 265 270
   Lys Leu Pro Gln Val Val Val Thr Pro His Leu Gly Ala Ser Thr Glu
   275 280 285
   Glu Ala Gln Asp Arg Ala Gly Thr Asp Val Ala Asp Ser Val Leu Lys
   290 295 300
   Ala Leu Ala Gly Glu Phe Val Ala Asp Ala Val Asn Val Ser Gly Gly
   305 310 315 320
   Arg Val Gly Glu Glu Val Ala Val Trp Met Asp Leu Ala Arg Lys Leu
   325 330 335
   Gly Leu Leu Ala Gly Lys Leu Val Asp Ala Ala Pro Val Ser Ile Glu
   340 345 350
   Val Glu Ala Arg Gly Glu Leu Ser Ser Glu Gln Val Asp Ala Leu Gly
   355 360 365
   Leu Ser Ala Val Arg Gly Leu Phe Ser Gly Ile Ile Glu Glu Ser Val
   370 375 380
   Thr Phe Val Asn Ala Pro Arg Ile Ala Glu Glu Arg Gly Leu Asp Ile
   385 390 395 400
   Ser Val Lys Thr Asn Ser Glu Ser Val Thr His Arg Ser Val Leu Gln
   405 410 415
   Val Lys Val Ile Thr Gly Ser Gly Ala Ser Ala Thr Val Val Gly Ala
   420 425 430
   Leu Thr Gly Leu Glu Arg Val Glu Lys Ile Thr Arg Ile Asn Gly Arg
   435 440 445
   Gly Leu Asp Leu Arg Ala Glu Gly Leu Asn Leu Phe Leu Gln Tyr Thr
   450 455 460
   Asp Ala Pro Gly Ala Leu Gly Thr Val Gly Thr Lys Leu Gly Ala Ala
   465 470 475 480
   Gly Ile Asn Ile Glu Ala Ala Ala Leu Thr Gln Ala Glu Lys Gly Asp
   485 490 495
   Gly Ala Val Leu Ile Leu Arg Val Glu Ser Ala Val Ser Glu Glu Leu
   500 505 510
   Glu Ala Glu Ile Asn Ala Glu Leu Gly Ala Thr Ser Phe Gln Val Asp
   515 520 525
   Leu Asp
   530